(19) Europäisches Patentamt
European Patent Office
Office européen des brevets

(11) Veröffentlichungsnummer: **0 222 334 B1**

# (12) EUROPÄISCHE PATENTSCHRIFT

(45) Veröffentlichungstag der Patentschrift: **04.09.91**

(51) Int. Cl.$^5$: **C07C 271/20**, C07C 271/24, C07C 271/28, D06M 15/568

(21) Anmeldenummer: **86115437.5**

(22) Anmeldetag: **07.11.86**

## (54) Perfluoralkylgruppen, von Epichlorhydrin abgeleiteten Gruppen und Dialkoholreste enthaltende Urethane, Verfahren zu ihrer Herstellung und ihre Verwendung.

(30) Priorität: **13.11.85 DE 3540147**

(43) Veröffentlichungstag der Anmeldung:
**20.05.87 Patentblatt 87/21**

(45) Bekanntmachung des Hinweises auf die Patenterteilung:
**04.09.91 Patentblatt 91/36**

(84) Benannte Vertragsstaaten:
**AT BE CH DE ES FR GB IT LI NL**

(56) Entgegenhaltungen:
**US-A- 3 952 075**

(73) Patentinhaber: **HOECHST AKTIENGESELLSCHAFT**
**Postfach 80 03 20**
**W-6230 Frankfurt am Main 80(DE)**

(72) Erfinder: **Wehowsky, Frank, Dr.**
**Talhauser Strasse 27**
**W-8269 Burgkirchen(DE)**
Erfinder: **Kleber, Rolf, Dr.**
**Am Trieb 41**
**W-6078 Neu-Isenburg(DE)**
Erfinder: **Jaeckel, Lothar**
**Ketteler Strasse 88**
**W-6093 Flörsheim am Main(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die Erfindung betrifft Perfluoralkylgruppen, von Epichlorhydrinabgeleiteten Gruppen und Dialkoholreste enthaltende Urethane. Die Erfindung betrifft ferner ein Verfahren zur Herstellung der neuen Urethanverbindungen und deren Verwendung.

In den US-Patentschriften 4 264 484, 4 340 749 und 4 468 527 sind Perfluoralkyl- und Epichlorhydringruppen enthaltende Urethane der nachstehenden Formel beschrieben (vgl. die Formeln I, V und VIII der genannten Patentschriften),

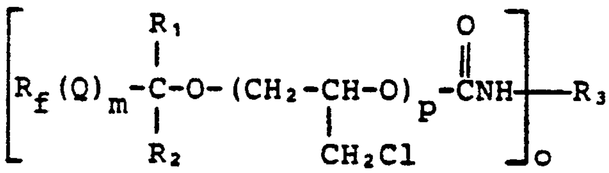

worin bedeuten

- $R_f$ einen fluoraliphatischen Rest,
- Q eine zweiwertige Gruppe, die frei von Epoxy-reaktionsfähigen und Isocyanat-reaktionsfähigen Gruppen ist, beispielsweise eine -CO-, -CONR-, $-SO_2NR-$, $-SO_2-$, $-C_nH_{2n}-$, $-C_6H_4-$, $-C_6H_3Cl-$ oder $-OC_2H_4$-Gruppe oder deren Kombinationen, wobei R ein Wasserstoffatom oder ein Alkylrest mit 1 bis 6 Kohlenstoffatomen und n 1 bis 20 ist,
- m 0 oder 1,
- $R_1$ ein Wasserstoffatom oder einen niederen Alkylrest,
- $R_2$ ein Wasserstoffatom, einen niederen Alkylrest oder einen Arylrest mit 6 bis 12 Kohlenstoffatomen, oder $R_1$ und $R_2$ miteinander unter Bildung einer aromatischen oder cycloaliphatischen Struktur verbunden sind,
- p eine Zahl mit einem kleinen Wert, beispielsweise 1 bis 5,
- o eine Zahl, die gleich der Zahl der Isocyanatgruppen im Isocyanat ist, beispielsweise 2 bis 5, und
- $R_3$ den Isocyanat-freien Rest eines organischen Polyisocyanates, wie 2,4-Toluylendiisocyanat.

Diese Urethane werden als öl- und wasserabweisende Mittel für Textilien empfohlen.

In den US-Patentschriften 3 721 700 und 3 952 075 sind Perfluoralkylgruppen und Dialkoholreste enthaltende Urethane beschrieben.

Die aus der US-Patentschrift 3 721 700 bekannten Urethane entsprechen der allgemeinen Formel

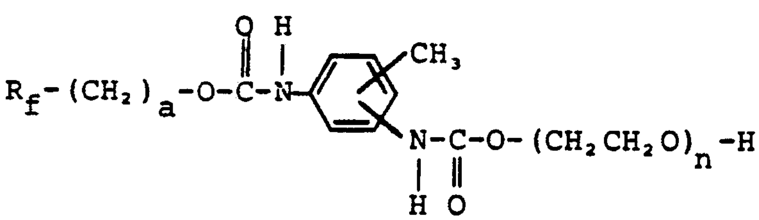

worin $R_f$ einen perfluorierten Alkylrest mit 3 bis 12 C-Atomen, n eine Zahl von 4 bis 70 und a 1 oder 2 ist.

Die aus der US-Patentschrift 3 952 075 bekannten Urethane entsprechen der allgemeinen Formel

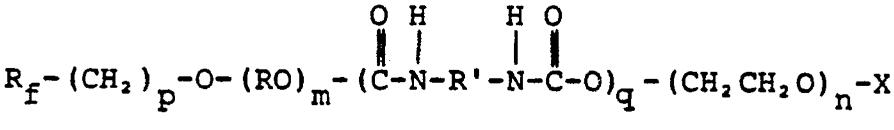

worin $R_f$ eine Perfluoralkylgruppe mit 4 bis 14 C-Atomen, R eine Alkylengruppe mit 2 bis 4 C-Atomen, R' eine Alkylen- oder Arylengruppe mit 2 bis 8 C-Atomen, X das Wasserstoffatom, $PO_3H_2$ oder $SO_3H$, p 1 bis 10, m und n 0 oder 1 bis 50 und q 0 oder 1 ist.

Die angegebenen Urethane werden in den beiden US-Patentschriften als öl- und wasserabweisende

Mittel für Textilien und als Dispergiermittel zur Herstellung von wäßrigen Dispersionen von polymeren, fluorhaltigen Ethyleniminderivaten beschrieben.

Es wurde nun überraschenderweise gefunden, daß perfluoralkylierte Urethane dann besonders hervorragende Eigenschaften im Hinblick auf die Ausrüstung von Textilien bezüglich Öl-, Wasser- und Schmutzabweisung besitzen, wenn sie neben mindestens einer Perfluoralkylgruppe auch noch Epichlorhydringruppen und eine Dialkoholkomponente im Molekül enthalten.
Die erfindungsgemäßen Urethan-Verbindungen entsprechen der nachstehenden allgemeinen Formel 1

$$\left[R_f-(CH_2)_x-O-(\underset{\underset{CH_2Cl}{|}}{CH_2-CH}-O)_y-\overset{\overset{O}{\|}}{C}-\overset{\overset{H}{|}}{N}\right]_m-A-\left[\overset{\overset{H}{|}}{N}-\overset{\overset{O}{\|}}{C}-(OR)_z-O-B\right]_n \quad (1),$$

worin bedeuten:

- $R_f$ eine Perfluoralkylgruppe mit 4 bis 20 C-Atomen, vorzugsweise 6 bis 16 C-Atomen, oder eine $R'_fSO_2NR_1$-Gruppe, in der $R'_f$ eine der Bedeutungen von $R_f$ hat und $R_1$ H oder eine Alkylgruppe mit 1 bis 4 C-Atomen ist,
- x eine ganze Zahl von 1 bis 4, vorzugsweise 2,
- y eine Zahl von 1 bis 10, vorzugsweise 1 bis 5,
- z eine Zahl von 1 bis 25, vorzugsweise 1 bis 20,
- m eine Zahl von 1 bis 2, und
- n eine Zahl von 1 bis 2, wobei die Summe aus m + n höchstens 3 ist,
- A eine der Gruppen entsprechend den nachstehenden Formeln 2 bis 10 (das sind isocyanatfreie Reste):

(2) 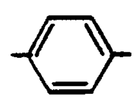

(3) 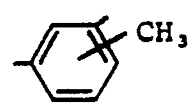

(4) 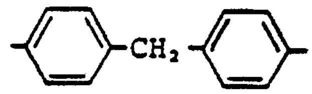

(5) 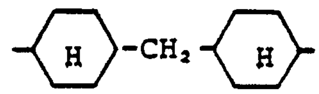

(6) 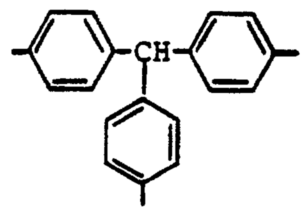

(7) $-(CH_2)_6-$

(8) 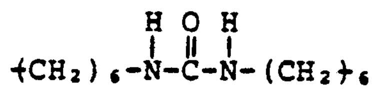

(9) 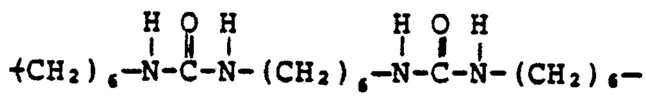

(10) 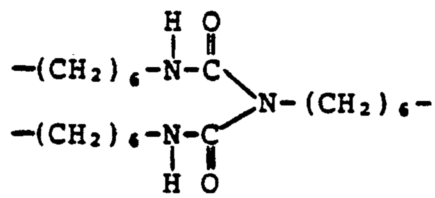

R eine Alkylengruppe mit 2 bis 6 C-Atomen, vorzugsweise 2 bis 4 C-Atomen, die Chlormethylethylengruppe oder eine Gruppe der Formel $-CH(CH_2OCH_2CH_2R_F)-CH_2-$ ($R_F$ = $C_4F_9$ bis $C_{20}F_{41}$, vorzugsweise $C_6F_{13}$ bis $C_{16}F_{33}$), wobei R auch mehrere dieser Bedeutungen haben kann, wenn z größer als 1 ist, und

B das Wasserstoffatom, eine Alkylgruppe mit 1 bis 4 C-Atomen oder eine Gruppe entsprechend der nachstehenden Formel 11

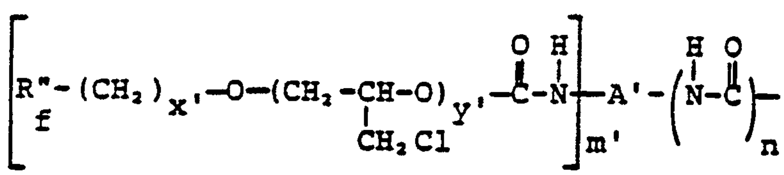 (11)

worin $R_f''$, x', y', m', n' und A' eine der Bedeutungen von $R_f$, x, y, m, n und A haben.

$R_f$ ist vorzugsweise die genannte Perfluoralkylgruppe. Diese Gruppe kann geradkettig oder verzweigt, vorzugsweise endverzweigt, sein. Die geradkettigen Perfluoralkylgruppen sind bevorzugt. Beim Perfluoralkylrest handelt es sich in der Regel um ein Gemisch von Perfluoralkylgruppen mit der obengenannten Anzahl von C-Atomen.

A ist vorzugsweise eine Toluylengruppe oder eine der drei Gruppen entsprechend den Formeln 8 bis 10 (diese drei Gruppen liegen in der Regel als Gemisch vor).

R ist vorzugsweise die Ethylen-, Propylen-, Butylen-, oder die Chlormethylethylengruppe: $-CH(CH_2Cl)-CH_2-$, wobei R auch mehrere dieser Bedeutungen haben kann, bei z >1; in diesem Fall ist R bevorzugt die Ethylen- und Propylengruppe.

B ist vorzugsweise Wasserstoff, eine Gruppe entsprechend der Formel 11, wobei A' eine Toluylengruppe oder eine der drei Gruppen entsprechend den Formeln 8 bis 10 ist, oder eine Alkylgruppe mit 1 bis 4 C-Atomen.

Die Herstellung der erfindungsgemäßen Perfluoralkylgruppen, Epichlorhydringruppen und Dialkoholreste

enthaltenden Urethane ergibt sich aus der allgemeinen Formel 1. Die Herstellung der neuen Urethane erfolgt vorzugsweise durch Reaktion eines fluorhaltigen Alkohols der Formel

$R_f(CH_2)_xOH$,

worin $R_f$ und x die obengenannte Bedeutung haben, mit Epichlorhydrin zum Perfluorhydroalkanol-Epichlorhydrin-Addukt beziehungsweise Perfluorsulfonamidoalkanol-Epichlorhydrin-Addukt der Formel

$$R_f(CH_2)_xO(CH_2\underset{\displaystyle CH_2Cl}{\underset{|}{C}}HO)_y-H \qquad \text{(Addukt 1)}$$

worin $R_f$, x und y die obengenannte Bedeutung haben, durch Reaktion des Adduktes 1 mit einem Di- oder Triisocyanat entsprechend einer der Gruppen gemäß Formeln 2 bis 10 zum Perfluoralkyl-Epichlorhydrin-Isocyanat-Addukt der Formel

$$\left[R_f(CH_2)_xO(CH_2\underset{\displaystyle CH_2Cl}{\underset{|}{C}}HO)_y-CONH\right]_m-A-(NCO)_n \qquad \text{(Addukt 2)}$$

worin $R_f$, x, y, m, und n die obengenannte Bedeutung haben, und durch Reaktion des Adduktes 2 mit einem Dialkohol (Diol) oder einem einseitig veretherten Diol der nachstehenden Formeln

$H(OR)_zOH$ beziehungsweise $H(OR)_zOB$

worin R und z die obengenannte Bedeutung haben und B das Wasserstoffatom oder eine Alkylgruppe mit 1 bis 4 C-Atomen ist, zu den angestrebten Perfluoralkyl- und Epichlorhydringruppen und Dialkoholreste enthaltenden Urethanen der beiden nachstehenden Formeln 12 und 13

$$\left[R_f(CH_2)_xO(CH_2\underset{\displaystyle CH_2Cl}{\underset{|}{C}}HO)_y-CONH\right]_m-A-\left[NHCO-(OR)_z-OH\right]_n \qquad (12)$$

$$\left[R_f(CH_2)_xO(CH_2\underset{\displaystyle CH_2Cl}{\underset{|}{C}}HO)_y-CONH\right]_m-A-\left[NHCO-(OR)_z-OB\right]_n \qquad (13),$$

worin $R_f$, x, y, z, m, n, A und B die obengenannte Bedeutung haben, und durch Reaktion eines Urethans der Formel 12 mit einer eine oder mehrere freie Isocyanatgruppen enthaltenden Isocyanatverbindung entsprechend der Gruppe gemäß Formel 11 zu den angestrebten Perfluoralkyl- und Epichlorhydringruppen und Dialkoholreste enthaltenden Urethanen der nachstehenden Formel 14

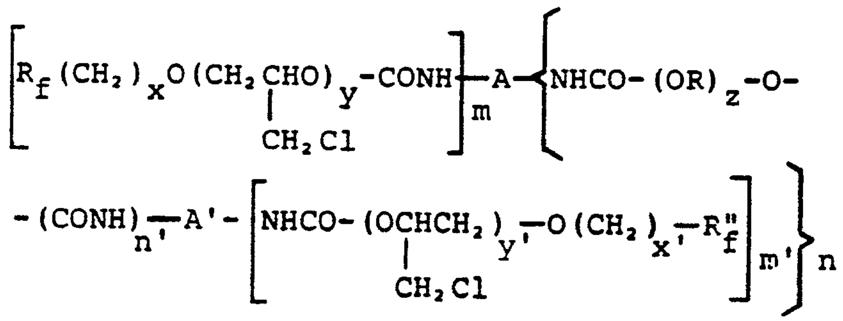 (14)

worin $R_f$, $R_f''$, x, x′, y, y′, z, m, m′, n, n′, A, A′ und R die obengenannte Bedeutung haben.

Im folgenden wird die Herstellung der erfindungsgemäßen Verbindungen im einzelnen beschrieben. Zur Herstellung des Adduktes 1, das ist das Perfluorhydroalkanol-Epichlorhydrin-Addukt beziehungsweise das Perfluorsulfonamidoalkanol-Epichlorhydrin-Addukt, wird vorzugsweise so vorgegangen, daß man den fluorhaltigen Alkohol, beispielsweise Perfluoralkylethanol beziehungsweise Perfluoralkylsulfonamidoethanol mit Epichlorhydrin (Siedepunkt bei Normalbedingungen 116 °C), gegebenenfalls in Gegenwart von Lewis-Säuren als Katalysator bei einer Temperatur von 30 bis 100 °C, vorzugsweise 40 bis 70 °C, umsetzt, wobei der fluorhaltige Alkohol und das Epichlorhydrin im Molverhältnis von etwa 1 : y eingesetzt werden (y hat die oben angegebene Bedeutung). Beim Perfluorhydroalkanol und beim Perfluorsulfonamidoalkanol handelt es sich bezüglich Perfluoralkylrest in der Regel um preisgünstige, im Handel erhältliche Gemische mit im wesentlichen 6 bis 16 C-Atomen. Die Art der Lewis-Säure ist nicht kritisch. Bevorzugt sind $BF_3$, Bortrifluoriddiethyletherat, $SnCl_4$, $SbCl_5$, $TiCl_4$, $FeCl_3$, $PF_5$ und/oder Dibutylzinndilaurat, wobei Bortrifluoriddiethyletherat besonders bevorzugt ist. Die Menge an Katalysator beträgt im allgemeinen 0,01 bis 5 Gew.-%, vorzugsweise 0,1 bis 1 Gew.-%, bezogen auf Perfluoralkylethanol. Die Umsetzung wird vorzugsweise unter Rühren und bei dem sich einstellenden Druck durchgeführt. Die Umsetzungsdauer liegt im Bereich von etwa 0,5 bis 7 Stunden. Es kann zweckmäßig sein ein Lösungsmittel einzusetzen. Bevorzugte Lösungsmittel sind halogenierte Kohlenwasserstoffe wie Tetrachlorkohlenstoff, Trichlorethylen, 1,2-Dichlorethan, Trichlorethan, Pentafluormonochlorethan und Trifluordichlorethan; Ketone wie Methylethylketon und Cyclohexanon; Ether wie Diisopropylether und Tetrahydrofuran; Dimethylformamid und N-Methylpyrrolidon. Die in Rede stehende Umsetzung läuft quantitativ ab. Im erhaltenen Reaktionsprodukt wird das gegebenenfalls verwendete Lösungsmittel abdestilliert, wobei auch gegebenenfalls vorhandene flüchtige Anteile wie nicht-umgesetztes Epichlorhydrin entfernt werden. Aus Zweckmäßigkeitsgründen kann man die Destillation auch unter Vakuum (Wasserstrahlvakuum) durchführen. Die als Katalysator eingesetzte Lewis-Säure, die bei der nachfolgenden Umsetzung mit Toluylendiisocyanat an sich nicht stört, kann mit Hilfe von alkalischen Mitteln, vorzugsweise mit Hilfe einer wäßrigen Natriumbicarbonat-Lösung oder einem Amin wie Triethylamin, ausgewaschen beziehungsweise neutralisiert werden. Das Addukt 1 stellt ein wachsartiges, gelb gefärbtes Produkt dar.

Zur Herstellung des Adduktes 2 wird vorzugsweise so vorgegangen, daß man das Addukt 1 mit einem Isocyanat entsprechend den Formeln 2 bis 10 bei einer Temperatur von 70 bis 150 °C, vorzugsweise 90 bis 130 °C, umsetzt, wobei die Addukt 1-Verbindung und das Isocyanat in dem Molverhältnis eingesetzt werden, das sich aus der angestrebten Bedeutung für m und n in der Formel für das Addukt 2 ergibt. Die Umsetzung wird vorzugsweise unter Rühren und bei dem sich einstellenden Druck durchgeführt und, sofern es zweckmäßig ist, beispielsweise zur Verkürzung der Reaktionszeit, in Gegenwart der obengenannten Lewis-Säure-Katalysatoren. Es können auch die obengenannten Lösungsmittel eingesetzt werden. Die Umsetzungsdauer liegt im Bereich von 1 bis 15 Stunden. Bei dem Isocyanat wird es sich oft um im Handel erhältliche Isocyanat-Gemische handeln. So besteht das Toluylendiisocyanat in der Regel aus etwa 80 Gew.-% 2,4-Toluylendiisocyanat und 20 Gew.-% 2,6-Toluylendiisocyanat. Auch die Isocyanate entsprechend den Gruppen gemäß Formel 8 bis 10 liegen in der Regel als Gemisch vor. Ein im Handel erhältliches und bevorzugtes derartiges Gemisch besteht aus den drei in Rede stehenden Isocyanaten, wobei das Isocyanat entsprechend der Formel 10 in einer Menge von mindestens 50 Gew.-%, bezogen auf die Mischung, vorhanden ist (in dieser Mischung ist also das Isocyanat entsprechend der Formel 10 die Hauptkomponente). Die Umsetzung des Adduktes 1 mit Isocyanat zum oben angegebenen Addukt 2 verläuft quantitativ. Die erhaltenen Produkte können gegebenenfalls gereinigt werden, beispielsweise können flüchtige Anteile abdestilliert werden. Das Addukt 2 stellt ein wachsartiges, gelb gefärbtes Produkt dar.

Zur Herstellung der erfindungsgemäßen Verbindungen der Formeln 12 und 13 wird vorzugsweise so

vorgegangen, daß man das Addukt 2 mit einem Diol oder einem einseitig veretherten Diol der oben angegebenen Art bei einer Temperatur von 70 bis 150 °C, vorzugsweise 90 bis 130 °C, umsetzt, wobei die Addukt 2-Verbindung und das Diol beziehungsweise der Diolmonoether in einem solchen Molverhältnis eingesetzt werden, daß die Molmenge an Diol beziehungsweise Diolether den in dem eingesetzten Addukt 2 vorhandenen freien Isocyanatgruppen entspricht. Die Umsetzung wird vorzugsweise unter Rühren und bei dem sich einstellenden Druck durchgeführt und, sofern es zweckmäßig ist, beispielsweise zur Verkürzung der Reaktionszeit, in Gegenwart der obengenannten Lewis-Säure-Katalysatoren. Es können auch die obengenannten Lösungsmittel eingesetzt werden. Die Umsetzungsdauer liegt im Bereich von 1 bis 15 Stunden. Die Umsetzung des Adduktes 2 mit dem Diol oder dem Diolmonoether zu den erfindungsgemäßen Verbindungen der Formeln 12 und 13 verläuft quantitativ. Das erhaltene Produkt kann gegebenenfalls gereinigt werden, beispielsweise können flüchtige Anteile abdestilliert werden. Die erfindungsgemäßen Verbindungen nach den Formeln 12 und 13 stellen wachsartige, gelb bis braun gefärbte Produkte dar.

Zur Herstellung der erfindungsgemäßen Verbindungen der Formel 14 wird vorzugsweise so vorgegangen, daß man eine Verbindung der Formel 12 mit einer Isocyanatverbindung entsprechend der Gruppe gemäß Formel 11 (vergleiche Addukt 2) bei einer Temperatur von 70 bis 150 °C, vorzugsweise 90 bis 130 °C, umsetzt, wobei die Verbindung nach Formel 12 und die Isocyanatverbindung in einem solchen Molverhältnis eingesetzt werden, daß die Molmenge an Isocyanatverbindung den in der eingesetzten Verbindung nach Formel 12 vorhandenen freien OH-Gruppen entspricht. Im übrigen gilt auch für die Herstellung dieser erfindungsgemäßen Verbindungen das, was oben über die Herstellung der erfindungsgemäßen Verbindungen nach den Formeln 12 und 13 gesagt worden ist. Die erfindungsgemäßen Verbindungen nach Formel 14 stellen ebenso wie jene nach den Formeln 12 und 13 wachsartige, gelb bis braun gefärbte Produkte dar.

Die erfindungsgemäßen Verbindungen stellen überraschend gute Textilbehandlungsmittel dar. Sie verleihen den Textilien vor allem eine hervorragende Hydrophobie und Oleophobie. Sie weisen ferner in einem hohen Ausmaß die Eigenschaft auf, den starken Beanspruchungen, denen die ausgerüsteten Textilien ausgesetzt werden, beispielsweise beim Verstrecken, Texturieren und insbesondere beim Färben und Waschen, ohne irgendeinen Verlust an Wirkung standzuhalten. Ein unerwarteter und besonders großer Vorteil der erfindungsgemäßen Verbindungen liegt darin, daß sie auch in üblichen Textilbehandlungspräparationen, beispielsweise in Spinnpräparationen, eingesetzt werden können und dabei ihre hervorragende Wirkung nicht verlieren.

Das Textilmaterial kann natürlicher und/oder synthetischer Natur sein. Es besteht vorzugsweise aus Polyamid, Polyester und/oder Polyacrylnitril, wobei Polyamid besonders bevorzugt ist. Das Textilmaterial kann in beliebiger Form vorliegen, so zum Beispiel als Faden, Faser, Garn, Gewebe, Gestrick, Gewirk, Teppich oder Vlies. Die Auftragsmenge an erfindungsgemäßer Verbindung wird so gewählt, daß auf dem Textilmaterial 0,02 bis 1 Gew.-% Fluor, vorzugsweise 0,04 bis 0,4 Gew.-% Fluor, vorhanden sind, berechnet aus der Menge an Fluor in der erfindungsgemäßen Verbindung; Gewichtsprozente bezogen auf das behandelte Textilmaterial. Die Behandlung des Textilmaterials mit den erfindungsgemäßen Urethanen erfolgt in der Regel entweder über die oben erwähnten Textilbehandlungspräparationen, denen die erfindungsgemäßen Urethane einverleibt worden sind, oder mit Hilfe von Lösungen, Emulsionen oder Dispersionen, die aus den Urethanen eigens bereitet worden sind. In den Lösungen, Emulsionen oder Dispersionen bzw. in den Textilbehandlungspräparationen liegen sie in der Regel in einer Konzentration von 5 bis 40 bzw. 0,5 bis 5 Gew.-% vor, vorzugsweise 8 bis 30 bzw. 1 bis 3 Gew.-%. Die Behandlung der Textilien mit den genannten Lösungen, Emulsionen oder Dispersionen wird nach üblichen Methoden durchgeführt, so zum Beispiel durch Sprühen, Tauchen, Foulardieren und dergleichen. Anschließend wird das getränkte Textilmaterial getrocknet und einer Wärmebehandlung unterworfen. Die Wärmebehandlung wird in der Regel in der Weise durchgeführt, daß das Textilmaterial auf eine Temperatur von 130 bis 200 °C erhitzt und bei dieser Temperatur 10 Sekunden bis 10 Minuten lang gehalten wird. Das mit den erfindungsgemäßen Urethanen ausgerüstete Textilmaterial besitzt die oben erwähnten hervorragenden Eigenschaften.

Die Erfindung wird nun an Beispielen noch näher erläutert.

## Erfindungsgemäße Verbindungen

### Beispiel 1

In einem mit einem Rührer, Rückflußkühler, Thermometer, Tropftrichter und Heizbad ausgestatteten Glaskolben wurden 527,9 g ( 1,0 mol) von einem im Handel erhältlichen Perfluoralkylethanolgemisch mit Perfluoralkyl = $C_6F_{13}$ bis $C_{12}F_{25}$ (OH-Zahl = 106), 380 ml 1,2,2-Trifluortrichlorethan ($CFCl_2$-$CF_2Cl$; Kp = 48 °C) als Lösungsmittel und 5,0 g Bortrifluoriddiethyletherat als Katalysator (das sind 1 Gew.-5 Katalysator, bezogen auf Perfluoralkylethanol) vorgelegt. Zu dieser Lösung wurden bei 45 °C 166,5 g ( 1,8 mol) Epichlorhydrin zugetropft, worauf 3 h lang bei der Siedetemperatur des Lösungsmittels gehalten wurde. Anschließend wurde das eingesetzte Lösungsmittel im Vakuum (Wasserstrahlvakuum) abdestilliert. Es lag ein wachsartiges gelb gefärbtes Produkt vor (Addukt 1). Dieses Addukt ist ein Perfluoralkylethanol-Epichlorhydrin-Addukt mit dem Molverhältnis Perfluoralkylethanol zu Epichlorhydrin gleich 1 : 1,8 (das heißt y in der Formel 1 ist 1,8). Nach der gleichen Arbeitsweise wurden auch die in den folgenden Beispielen eingesetzten Addukte 1 bereitet.

Nachstehend ist der Ansatz für die weiteren Umsetzungen zur Herstellung der erfindungsgemäßen Verbindungen zusammengefaßt. Diese Umsetzungen wurden jeweils in einem mit einem Rührer, Rückflußkühler mit Trockenrohr, Thermometer und Heizbad ausgestatteten Glaskolben durchgeführt.

Ansatz:

100,0 g (0,144 mol) Addukt 1

25,1 g (0,144 mol) Toluylendiisocyanat, und zwar ein Gemisch aus etwa 80 Gew.-%, 2,4- und etwa 20 Gew.-% 2,6-Toluylendiisocyanat (das ist ein Handelsprodukt)

82,5 g (0,144 mol) Hexaepichlorhydrin:

$$H(O\underset{\displaystyle |}{C}HCH_2)_6\text{-}OH. \quad \text{mit } CH_2Cl \text{ am C}$$

Addukt 1-Verbindung, Toluylendiisocyanat und Hexaepichlorhydrin wurden also im Molverhältnis 1 : 1 : 1 eingesetzt.

Durchführung:

Die Addukt 1-Verbindung und das Toluylendiisocyanat wurden vorgelegt. Die Mischung wurde auf 100 bis 110 °C erhitzt, bei dieser Temperatur 3 Stunden lang unter Rühren gehalten und anschließend abgekühlt (1. Reaktionsstufe). Das erhaltene Perfluoralkylethanol-Epichlorhydrin-Toluylendiisocyanat-Addukt war ein wachsartiges, gelb gefärbtes Produkt. Zu diesem im Glaskolben befindlichen Produkt wurde das Hexaepichlorhydrin zugegeben. Die Mischung wurde auf 100 bis 110 °C erhitzt, bei dieser Temperatur 3 Stunden lang unter Rühren gehalten und anschließend abgekühlt (2. Reaktionsstufe). Es wurde ein wachsartiges, braun gefärbtes Produkt erhalten (Ausbeute 206,1 g, das sind 99,3 Gew.-% der Theorie). Die Bruttozusammensetzung der erhaltenen erfindungsgemäßen Verbindung entspricht der in der Tabelle nach den Beispielen angegebenen Formel mit der laufenden Nummer B1.

Beispiel 2

Ansatz:

69,4 g (0,1 mol) Addukt 1 gemäß Beispiel 1

17,4 g (0,1 mol) Toluylendiisocyanat gemäß Beispiel 1

20,3 g (0,1 mol) Diepichlorhydrin.

Addukt 1-Verbindung, Toluylendiisocyanat und Diepichlorhydrin wurden also im Molverhältnis 1 : 1 : 1 eingesetzt.

Durchführung:

Sie erfolgte analog Beispiel 1. Es wurde ein wachsartiges, braun gefärbtes Produkt erhalten (Ausbeute: 105,2 g, das sind 98,1 Gew.-% der Theorie). Die Bruttozusammensetzung der erhaltenen erfindungsgemäßen Verbindung entspricht der Formel mit der laufenden Nummer B2 in der genannten Tabelle.

Beispiel 3

Ansatz:

133,2 g (0,2 mol) Addukt 1 gemäß Beispiel 1

54,4 g (0,2 mol) Diphenylmethan-4,4'-diisocyanat (das ist ein Handelsprodukt)

114,6 g (0,2 mol) Hexaepichlorhydrin.

Addukt 1-Verbindung, Diphenylmethandiisocyanat und Hexaepichlorhydrin wurden also im Molverhältnis 1 : 1 : 1 eingesetzt.

Durchführung:

Das Diisocyanat wurde vorgelegt, durch Erhitzen aufgeschmolzen und zu dem aufgeschmolzenen Diisocyanat wurde unter Rühren die geschmolzene Addukt 1-Verbindung zugegeben. Die Mischung wurde auf 110

bis 120 °C erhitzt, bei dieser Temperatur 3 Stunden lang unter Rühren gehalten und anschließend abgekühlt (1. Reaktionsstufe). Das erhaltene Perfluoralkylethanol-Epichlorhydrin-Diphenylmethandiisocyanat-Addukt war ein wachsartiges, gelb gefärbtes Produkt. Zu diesem im Glaskolben befindlichen Produkt wurde das Hexaepichlorhydrin zugegeben.

Die Mischung wurde auf 110 bis 120 °C erhitzt, bei dieser Temperatur 4 Stunden lang unter Rühren gehalten und anschließend abgekühlt (2. Reaktionsstufe). Es wurde ein wachsartiges, braun gefärbtes Produkt erhalten (Ausbeute) 298,6 g, das sind 98,8 Gew.-% der Theorie). Die Bruttozusammensetzung der erhaltenen erfindungsgemäßen Verbindung entspricht der Formel mit der laufenden Nummer B3 in der genannten Tabelle.

Beispiel 4

Ansatz:
310,0 g (0,442 mol) Addukt 1 gemäß Beispiel 1
377,0 g (0,221 mol) Triphenylmethan-4,4', 4''-triisocyanat
24,4 g (0,221 mol) 1-Chlormethyl-1,2-dihydroxyethan:

$CH_2Cl$
|
$HOCHCH_2-OH$

Addukt 1-Verbindung, Triisocyanat und die Diolverbindung wurden also im Molverhältnis 2 : 1 : 1 eingesetzt.
Durchführung:
Sie erfolgte analog Beispiel 3, wobei in der ersten Reaktionsstufe 5 Stunden lang bei 110 bis 120 °C gehalten, anstelle des Hexaepichlorhydrins das obengenannte Chlorpropandiol zugegeben und in der zweiten Reaktionsstufe 8 Stunden lang bei 120 bis 130 °C gehalten wurde. Es wurde ein wachsartiges, braun gefärbtes Produkt erhalten (Ausbeute 709 g, das sind 99,7 Gew.-% der Theorie). Die Bruttozusammensetzung der erhaltenen erfindungsgemäßen Verbindung entspricht der Formel mit der laufenden Nummer B4 in der genannten Tabelle.

Beispiel 5

Ansatz:
150,0 g (0,225 mol) Addukt 1 gemäß Beispiel 1
115,3 g (0,150 mol) Triisocyanat entsprechend Formel 10, und zwar ein Gemisch aus den drei Isocyanaten, entsprechend den Formeln 8, 9 und 10 mit dem Triisocyanat als Hauptbestandteil (das ist ein Handelsprodukt)
129,0 g (0,225 mol) Hexaepichlorhydrin.
Addukt 1-Verbindung, Isocyanat und Hexaepichlorhydrin wurden also im Molverhältnis 3 : 2 : 3 eingesetzt.
Durchführung:
Das Isocyanat und 1,2 g Dibutylzinndilaurat als Katalysator (das sind 1 Gew.-%, bezogen auf die eingesetzte Menge an Isocyanat) wurden vorgelegt, durch Erhitzen aufgeschmolzen und zu der aufgeschmolzenen Mischung wurde unter Rühren die geschmolzene Addukt 1-Verbindung zugegeben. Die Mischung wurde bei 65 °C 4 Stunden lang unter Rühren gehalten und anschließend abgekühlt (1. Reaktionsstufe). Das erhaltene Perfluoralkylethanol-Epichlorhydrin-Isocyanat-Addukt war ein wachsartiges, gelb gefärbtes Produkt. Zu diesem im Glaskolben befindlichen Produkt wurde das Hexaepichlorhydrin zugegeben. Die Mischung wurde auf 100 bis 110 °C erhitzt, bei dieser Temperatur 4 Stunden lang unter Rühren gehalten und anschließend abgekühlt (2. Reaktionsstufe). Es wurde ein wachsartiges, braun gefärbtes Produkt erhalten (Ausbeute 371,5 g, das sind 94,2 Gew.-% der Theorie). Die Bruttozusammensetzung der erhaltenen erfindungsgemäßen Verbindung entspricht der Formel B5.

Beispiel 6

Ansatz:
105,6 g (0,13 mol) Perfluoralkylethanol-Epichlorhydrin-Addukt mit dem Molverhältnis Perfluoralkylethanol zu Epichlorhydrin gleich 1 : 3 (das heißt y in der Formel 1 ist 3) als Addukt 1. Das Perfluoralkylethanol war ein im Handel erhältliches Gemisch mit Perfluoralkyl = $C_8F_{17}$ bis $C_{16}F_{33}$ (OH-Zahl = 69 ).

34,4 g (0,13 mol) Dicyclohexylmethan-4,4'-diisocyanat (das ist ein Handelsprodukt)
82,3 g (0,13 mol) Polytetrahydrofuran mit der Molmasse 650: $H(OC_4H_8)_{8,5}$-OH.
Addukt 1-Verbindung, Dicyclohexylmethandiisocyanat und das Polytetrahydrofuran (Diol) wurden also im Molverhältnis 1 : 1 : 1 eingesetzt.
Durchführung:
Sie erfolgte analog Beispiel 3, wobei anstelle des Hexaepichlorhydrins das obengenannte Polytetrahydrofuran zugegeben wurde. Es wurde ein wachsartiges, braun gefärbtes Produkt erhalten (Ausbeute: 220,5 g, das sind 99,2 Gew.-% der Theorie). Die Bruttozusammensetzung der erhaltenen erfindungsgemäßen Verbindung entspricht der Formel B6.

Beispiel 7

Ansatz:
81,3 g (0,10 mol) Addukt 1 gemäß Beispiel 6
28,5 g (0,05 mol) Triisocyanat gemäß Beispiel 5
31,7 g (0,05 mol) Polytetrahydrofuran gemäß Beispiel 6.
Addukt 1, Triisocyanat und Polytetrahydrofuran wurden also im Molverhältnis 2 : 1 : 1 eingesetzt.
Durchführung:
Sie erfolgte analog Beispiel 1, wobei anstelle des Hexaepichlorhydrins das obengenannte Polytetrahydrofuran zugegeben und in der zweiten Reaktionsstufe 7 Stunden lang bei 100 bis 110 °C gehalten wurde. Es wurde ein wachsartiges, braun gefärbtes Produkt erhalten (Ausbeute: 138,4 g, das sind 98,2 Gew.-% der Theorie). Die Bruttozusammensetzung der erhaltenen erfindungsgemäßen Verbindung entspricht der Formel B7.

Beispiel 8

Ansatz:
97,5 g (0,12 mol) Addukt 1 gemäß Beispiel 6
34,2 g (0,06 mol) Triisocyanat gemäß Beispiel 5
58,0 g (0,06 mol) einseitig verethertes Diol der Formel

$$H(OCH(CH_3)CH_2)_{10}-OC_4H_9$$

Addukt 1, Triisocyanat und Dekapropylenglykolmono-n-butylether wurden also im Molverhältnis 2 : 1 : 1 eingesetzt.
Durchführung:
Sie erfolgte analog Beispiel 1, wobei in der ersten Reaktionsstufe 6 Stunden lang bei 100 bis 110 °C gehalten, anstelle des Hexaepichlorhydrins der obengenannte Diolmonoether zugegeben und in der zweiten Reaktionsstufe 8 Stunden lang bei 100 bis 110 °C gehalten wurde. Es wurde ein wachsartiges, braun gefärbtes Produkt erhalten (Ausbeute 184 g, das sind 97,2 Gew.-% der Theorie). Die Bruttozusammensetzung der erhaltenen erfindungsgemäßen Verbindung entspricht der Formel B8.

Beispiel 9

Ansatz:
81,3 g (0,10 mol) Addukt 1 gemäß Beispiel 6
28,5 g (0,05 mol) Triisocyanat gemäß Beispiel 5
54,7 g (0,05 mol) einseitig verethertes Diol der Formel

$$H(OCH(CH_3)CH_2)_{10}-(OCH_2CH_2)_{10}-OC_4H_9$$

Addukt 1, Triisocyanat und Deka(Propylen-Ethylen)glykolmono-n-butylether wurden also im Molverhältnis 2 :

1 : 1 eingesetzt.

Durchführung:

Sie erfolgte analog Beispiel 5, wobei in der ersten Reaktionsstufe 6 Stunden lang bei 100 bis 110 °C gehalten, anstelle des Hexaepichlorhydrins der obengenannte Diolmonoether zugegeben und in der zweiten Reaktionsstufe 10 Stunden lang bei 100 bis 110 °C gehalten wurde. Es wurde ein wachsartiges, braun gefärbtes Produkt erhalten (Ausbeute 161,7 g, das sind 98,3 Gew.-% der Theorie). Die Bruttozusammensetzung der erhaltenen erfindungsgemäßen Verbindung entspricht der Formel B9.

Beispiel 10

Ansatz:

162,6 g (0,20 mol) Addukt 1 gemäß Beispiel 6

57,0 g (0,10 mol) Triisocyanat gemäß Beispiel 5

64,1 g (0,05 mol) Polytetrahydrofuran gemäß Beispiel 6.

Das Molverhältnis der eingesetzten Verbindungen war also 4 : 2 : 1.

Durchführung:

Die Addukt 1-Verbindung und das Triisocyanat wurden vorgelegt. Die Mischung wurde auf 100 bis 110 °C erhitzt und bei dieser Temperatur 3 Stunden lang unter Rühren gehalten (1. Reaktionsstufe). Zu dem erhaltenen wachsartigen, gelb gefärbten Perfluoralkylethanol-Epichlorhydrin-Isocyanat-Addukt wurde das Polytetrahydrofuran zugegeben. Die Mischung wurde 4 Stunden lang bei 100 bis 110 °C unter Rühren gehalten und anschließend abgekühlt (2. Reaktionsstufe). Es wurde ein wachsartiges, braun gefärbtes Produkt erhalten (Ausbeute 278,2 g, das sind 98,1 Gew.-% der Theorie). Die Bruttozusammensetzung der erhaltenen erfindungsgemäßen Verbindung entspricht der Formel B10.

Beispiel 11

Ansatz:

81,3 g (0,10 mol) Addukt 1 gemäß Beispiel 6

28,5 g (0,05 mol) Triisocyanat gemäß Beispiel 5

3,1 g (0,05 mol) Monoethylenglykol

49,3 g (0,05 mol) Verbindung der nachstehenden Formel

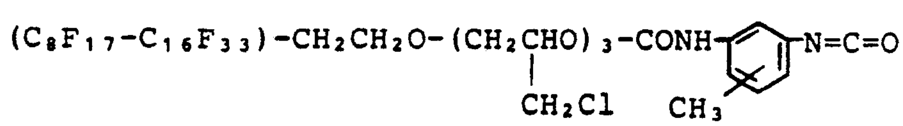

Das Molverhältnis der eingesetzten Verbindungen war also 2 : 1 : 1 : 1.

Durchführung:

Sie erfolgte analog Beispiel 10, wobei in der 2. Reaktionsstufe des Monoethylenglykol zugegeben wurde. Zu dem erhaltenen wachsartigen, braun gefärbten Produkt (das der im Beispiel 7 hergestellten erfindungsgemäßen Verbindung entspricht) wurden die 0,05 mol des oben angegebenen Perfluoralkylethanol-Epichlorhydrin-Isocyanat-Adduktes zugegeben, das erhalten worden war durch Umsetzung von 0,1 mol Addukt 1-Verbindung mit 0,05 mol Toluylendiisocyanat. Die Mischung wurde 3 Stunden lang bei 100 bis 110 °C unter Rühren gehalten und anschließend abgekühlt (3. Reaktionsstufe). Es wurde ein wachsartiges, braun gefärbtes Produkt erhalten (Ausbeute 160,7 g, das sind 99,1 Gew.-% der Theorie). Die Bruttozusammensetzung der erhaltenen erfindungsgemäßen Verbindung entspricht der Formel B11).

Beispiel 12

Ansatz:

81,3 g (0,10 mol) Addukt 1 gemäß Beispiel 6

28,5 g (0,05 mol) Triisocyanat gemäß Beispiel 5

5,8 g (0,05 mol) 1,6-Hexandiol.

Das Molverhältnis der eingesetzten Verbindungen war also 2 : 1 : 1.

Durchführung:

Sie erfolgte analog Beispiel 7, wobei anstelle des Polytetrahydrofurans das obengenannte 1,6-Hexandiol

eingesetzt wurde. Es wurde ein wachsartiges, braun gefärbtes Produkt erhalten (Ausbeute 114,3 g, das sind 98,8 Gew.-% der Theorie). Die Bruttozusammensetzung der erhaltenen erfindungsgemäßen Verbindung entspricht der Formel B12.

Beispiel 13

Ansatz:
162,6 g (0,20 mol) Addukt 1 gemäß Beispiel 6
57,0 g (0,10 mol) Triisocyanat gemäß Beispiel 5
9,0 g (0,10 mol) 1,4-Butandiol.
Das Molverhältnis der eingesetzten Verbindungen war also 2 : 1 : 1.
Durchführung:
Sie erfolgte analog Beispiel 12. Es wurde ein wachsartiges, ocker gefärbtes Produkt erhalten (Ausbeute 226,5 g, das sind 99,1 Gew.-% der Theorie). Die Bruttozusammensetzung der erhaltenen erfindungsgemäßen Verbindung entspricht der Formel B13.

Beispiel 14

Ansatz:
162,6 g (0,20 mol) Addukt 1 gemäß Beispiel 6
57,0 g (0,10 mol) Triisocyanat gemäß Beispiel 5
27,1 g (0,50 mol) fluorierte Etherdiol-Verbindung der Formel:

$$\begin{array}{l} HOCH-CH_2OH \\ \quad\ | \\ \quad CH_2OCH_2CH_2C_8F_{17}. \end{array}$$

Das Molverhältnis der eingesetzten Verbindungen war also 4 : 2 : 1.
Durchführung:
Die Addukt 1-Verbindung und das Triisocyanat wurden vorgelegt. Die Mischung wurde auf 110 °C erhitzt und bei dieser Temperatur 4 Stunden lang unter Rühren gehalten, worauf 4 Tropfen Dibutylzinndilaurat zugegeben und weitere 3 Stunden lang bei 110 °C unter Rühren gehalten wurde (1. Reaktionsstufe, vergleiche Beispiel 10). Zu dem erhaltenen wachsartigen, gelb gefärbten Perfluoralkylethanol-Epichlorhydrin-Isocyanat-Addukt wurde die fluorierte Etherdiol-Verbindung gelöst in 55 g Adipinsäure-di-n-butylester,zugegeben. Die Mischung wurde 5 Stunden lang bei 110 °C unter Rühren gehalten und anschließend abgekühlt (2. Reaktionsstufe). Es wurde ein wachsartiges, braun gefärbtes Produkt erhalten (Ausbeute 300 g, das sind 99,5 Gew.-% der Theorie). Die Bruttozusammensetzung der erhaltenen erfindungsgemäßen Verbindung entspricht der Formel B 14.

T A B E L L E

| Nr. | Chemische Formeln der erfindungsgemäßen Verbindungen nach den Beispielen 1 bis 14 |
|---|---|
| B1 | 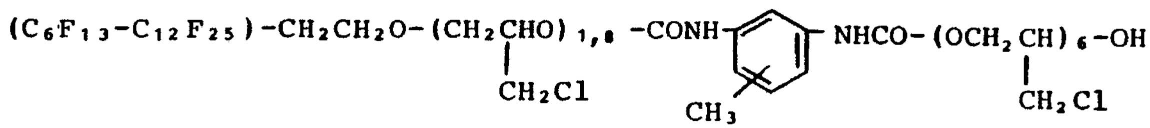 |
| B2 | 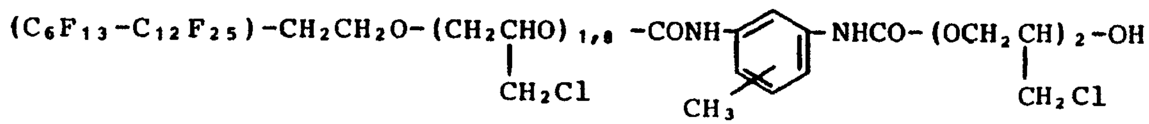 |
| B3 | 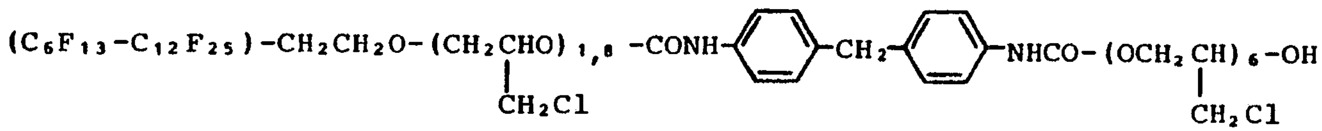 |
| B4 | 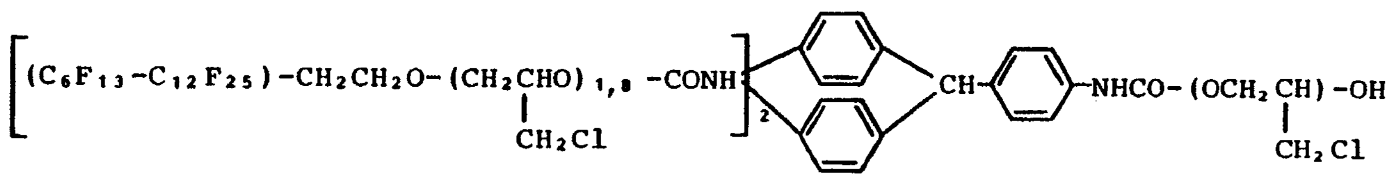 |
| B5 | 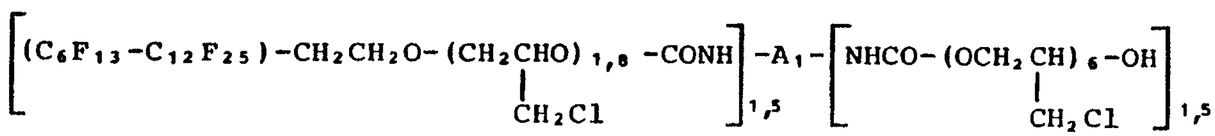 |

Fortsetzung der TABELLE

| Nr. | Chemische Formeln der erfindungsgemäßen Verbindungen nach den Beispielen 1 bis 14 |
|---|---|
| B6 | 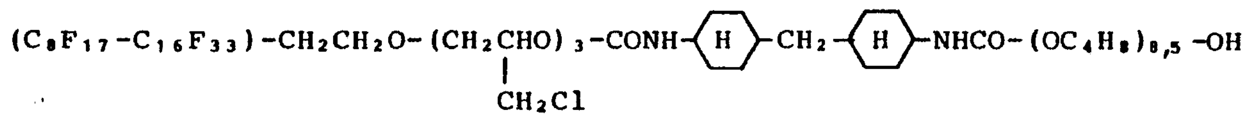 |
| B7 | $[(C_8F_{17}-C_{16}F_{33})-CH_2CH_2O-(CH_2CHO)_3-CONH]_2-A_2-NHCO-(OC_4H_8)_{8,5}-OH$<br>$CH_2Cl$ (an $CH_2\underline{C}HO$) |
| B8 | $[(C_8F_{17}-C_{16}F_{33})-CH_2CH_2O-(CH_2CHO)_3-CONH]_2-A_3-NHCO-(OCHCH_2)_{10}-OC_4H_9$<br>$CH_2Cl$ (an $CH_2\underline{C}HO$); $CH_3$ (an $O\underline{C}HCH_2$) |
| B9 | $[(C_8F_{17}-C_{16}F_{33})-CH_2CH_2O-(CH_2CHO)_3-CONH]_2-A_4-NHCO-(OCHCH_2)_{10}(OC_2H_4)_{10}-OC_4H_9$<br>$CH_2Cl$ (an $CH_2\underline{C}HO$); $CH_3$ (an $O\underline{C}HCH_2$) |
| B10 | $[(C_8F_{17}-C_{16}F_{33})-CH_2CH_2O-(CH_2CHO)_3-CONH]_2-A_5-NHCO-(OC_4H_8)_{8,5}-OB_1$<br>$CH_2Cl$ (an $CH_2\underline{C}HO$) |
| B11 | $[(C_8F_{17}-C_{16}F_{33})-CH_2CH_2O-(CH_2CHO)_3-CONH]_2-A_6-NHCO-(OC_2H_4)-OB_2$<br>$CH_2Cl$ (an $CH_2\underline{C}HO$) |

Fortsetzung der T A B E L L E

| Nr. | Chemische Formeln der erfindungsgemäßen Verbindungen nach den Beispielen 1 bis 14 |
|---|---|
| B12 | $\left[(C_8F_{17}-C_{16}F_{33})-CH_2CH_2O-(CH_2CHO)_3-CONH\right]_2-A_7-NHCO-(OC_6H_{12})-OH$<br>(CHO branch: $CH_2Cl$) |
| B13 | $\left[(C_8F_{17}-C_{16}F_{33})-CH_2CH_2O-(CH_2CHO)_3-CONH\right]_2-A_8-NHCO-(OC_4H_8)-OH$<br>(CHO branch: $CH_2Cl$) |
| B14 | $\left[(C_8F_{17}-C_{16}F_{33})-CH_2CH_2O-(CH_2CHO)_3-CONH\right]_2-A_9-NHCO-(OCHCH_2)-OB_3$<br>(CHO branch: $CH_2Cl$; OCHCH₂ branch: $CH_2OC_2H_4C_8F_{17}$) |

In den Formeln B5 und B7 bis B14 steht $A_1$ bis $A_9$ für

$$-(CH_2)_6NHCO\diagdown$$
$$N-(CH_2)_6- \quad \text{(vgl. obengenannte Formel 10).}$$
$$-(CH_2)_6NHCO\diagup$$

In den Formeln B10, B14 und B11 steht

$B_1$ für 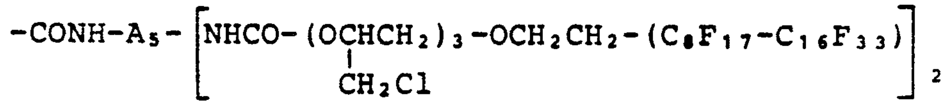

$B_3$ für 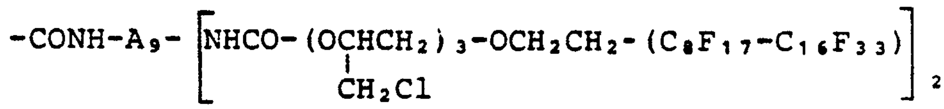

und

$B_2$ für 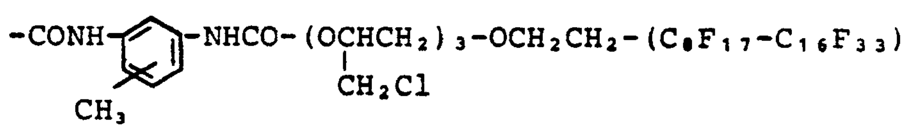

Verwendung der erfindungsgemäßen Verbindungen

Beispiele I bis XIV

In diesen Beispielen wurden die erfindungsgemäßen Verbindungen B1 bis B14 getestet.

In den Beispielen I bis VI wurden die Verbindungen B1 bis B6 mit Hilfe einer eigens bereiteten Lösung getestet, die jeweils aus ca. 1,2 g erfindungsgemäßer Verbindung und 250 g Aceton bestand (acetonische Lösung der Flotte). Mit jeder der sechs acetonischen Flotten wurde ein jeweils gleiches Gewebe aus Polyamid-6-Filamenten behandelt, um soviel von der erfindungsgemäßen Verbindung auf das Gewebe aufzubringen, daß 0,05 Gew.-% Fluor auf dem Gewebe vorlagen (Fluorauflage), Gewichtsprozente bezogen auf das Gewicht des Gewebes. Dazu wurde das Gewebe in üblicher Weise in die Flotte getaucht, mit einem Foulard eine Flottenaufnahme von 30 bis 40 Gew.-% eingestellt, das aceton-feuchte Gewebe zunächst luftgetrocknet und dann 30 Sekunden lang bei einer Temperatur von 200 °C gehalten (Wärmebehandlung, auch Kondensation genannt). Nach dieser Behandlung lagen sechs Gewebe mit den erfindungsgemäßen Verbindungen B1 bis B6 vor, wobei auf jedem Gewebe eine Fluorauflage von 0,05 Gew.-% vorhanden war, Gewichtsprozent bezogen auf das Gewicht des Gewebes.

An den sechs Geweben wurden die Ölabweisung (Oleophobie) nach der AATCC-Prüfnorm 118 - 1966 und die Wasserabweisung (Hydrophobie) nach DIN 53 888 - 1965 geprüft, und zwar nach der beschriebenen Kondensation und nach einer dreistündigen Behandlung der kondensierten Gewebe mit einer alkalischen Kochwäsche. Bei dieser Behandlung wurden die einzelnen Gewebe in üblicher Weise 3 Stunden lang in einer alkalischen Waschflüssigkeit gekocht und anschließend getrocknet; die Waschflüssigkeit bestand aus 1 l Wasser, 1 g Trinatriumphosphat und 2 g eines Fettsäurepolyglykolesters, der durch Oxethylierung von Butandiol-1,4 mit 15 mol Ethylenoxid und anschließender Veresterung des Oxethylats mit 1 mol Ölsäure erhalten worden ist.

In den Beispielen VII bis XIV wurden die erfindungsgemäßen Verbindungen B7 bis B14 mit Hilfe einer üblichen Spinnpräparation für Polyamidfasern getestet, die jeweils ca. 150 g erfindungsgemäße Verbindung pro 1000 g Spinnpräparation enthielt (die Spinnpräparation bestand also aus Wasser als Hauptkomponente, den üblichen ethoxylierten Fettalkoholen und langkettigen Aminoxiden als Präparationsmittel und ca. 15 Gew.-% erfindungsgemäßer Verbindung). Mit jeder der acht Spinnpräparationen wurden jeweils gleiche Polyamid-6-Filamente behandelt, um soviel von der erfindungsgemäßen Verbindung und dem Präparationsmittel auf die Filamente aufzubringen, daß 0,05 Gew.-% Fluor und 1 Gew.-% Präparationsmittel auf den Filamenten vorlangen, Gewichtsprozente jeweils bezogen auf das Gewicht der Filamente. Dazu wurden die Filamente in üblicher Weise durch die Spinnpräparation gezogen, getrocknet und 30 Sekunden lang bei einer Temperatur von 200 °C gehalten (Wärmebehandlung, Kondensation). Aus den so behandelten Filamenten wurde jeweils ein Gewebe hergestellt. Es lagen acht Gewebe mit den erfindungsgemäßen Verbindungen B7 bis B14 vor, wobei auf jedem Gewebe eine Fluorauflage von 0,05 Gew.-% und eine Präparationsmittelauflage von 1 Gew.-% vorhanden waren, Gewichtsprozente jeweils bezogen auf das Gewicht des Gewebes.

An den acht Geweben wurden, wie oben beschrieben, die Ölabweisung und die Wasserabweisung geprüft.

Die Ergebnisse aus den Beispielen I bis XIV sind nachstehend zusammengefaßt.

| Beispiel und getestete Verbindungen | Ölabweisung | | Wasserabweisung | |
|---|---|---|---|---|
| | nach der Konden-sation | nach der Koch-wäsche | nach der Konden-sation | nach der Koch-wäsche |
| I /B1 | 6 | 5 | 5 | 5 |
| II /B2 | 5 | 5 | 5 | 5 |
| III/B3 | 5 | 5 | 5 | 5 |
| IV /B4 | 6 | 5 | 5 | 4 |
| V /B5 | 5 | 4 | 5 | 5 |
| VI /B6 | 6 | 5 | 5 | 4 |
| VII/B7 | 6 | 4 | 5 | 5 |
| VIII/B8 | 5 | 4 | 4 | 4 |
| IX /B9 | 5 | 4 | 4 | 4 |
| X /B10 | 5 | 4 | 5 | 4 |
| XI /B11 | 6 | 5 | 4 | 4 |
| XII /B12 | 5 | 4 | 4 | 4 |
| XIII/B13 | 5 | 4 | 4 | 4 |
| XIV /B14 | 6 | 4 | 4 | 4 |

Im folgenden wird der AATCC-Test 118 - 1966 (American Association of Textile Chemists and Colorists) und DIN 53 888 - 1965 (Deutsche Industrie Norm) beschrieben:
Zur Bestimmung des Ölabweisungswertes nach AATCC-Test 118 - 1966 werden bekanntlich drei Tropfen von einer bestimmten Testflüssigkeit (siehe unten) vorsichtig auf das zu prüfende Textilmaterial aufgelegt. Einwirkungszeit: 30 Sekunden. Es wird der Wert angegeben, bei dem noch keine augenscheinliche Benetzung des Gewebes unter den Tropfen (nach abgelaufener Einwirkungszeit) hervorgerufen worden ist:

| Testflüssigkeit | Ölabweisungswert |
|---|---|
| Paraffinöl | 1 |
| Praffinöl : n-Hexadecan = 65:35 | 2 |
| n-Hexadecan | 3 |
| n-Tetradecan | 4 |
| n-Dodecan | 5 |
| n-Decan | 6 |
| n-Octan | 7 |
| n-Heptan | 8 |

Ein Ölabweisungswert von 1 bedeutet den schlechtesten und ein Ölabweisungswert von 8 den besten Abweisungseffekt.

Zur Bestimmung des Wasserabweisungswertes nach DIN 53 888 - 1965 werden bekanntlich die zu prüfenden Textilien unter standardisierten Bedingungen beregnet, wobei gleichzeitig die Unterseite der Textilprobe mechanisch gerieben wird. Es wird der Wasserabperleffekt visuell mit den Noten 1 bis 5 beurteilt, wobei Note 1 den schlechtesten und Note 5 den besten Abperleffekt bedeutet.

Die Testergebnisse zeigen, daß mit den erfindungsgemäßen Urethanen eine sehr hohe Öl- und Wasserabweisung erreicht wird, und daß die erfindungsgemäßen Urethane auch Textilbehandlungspräparationen zugesetzt werden können.

## Patentansprüche

**Patentansprüche für folgende Vertragsstaaten : BE, CH, DE, FR, GB, IT, LI, NL**

**1.** Perfluoralkylgruppen, von Epichlorhydrinabgeleiteten Gruppen und Dialkoholreste enthaltende Urethane der nachstehenden allgemeinen Formel 1

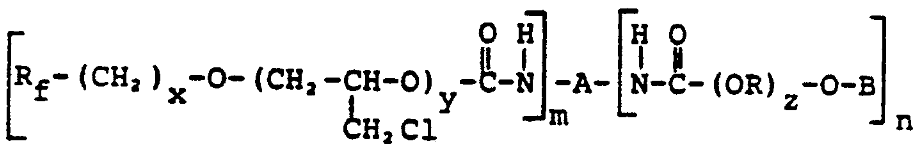 (1),

worin bedeuten:

$R_f$ eine Perfluoralkylgruppe mit 4 bis 20 C-Atomen oder eine $R_f'$ $SO_2NR_1$-Gruppe, in der $R_f'$ eine der Bedeutungen von $R_f$ hat und $R_1$ H oder eine Alkylgruppe mit 1 bis 4 C-Atomen ist,
x eine ganze Zahl von 1 bis 4,
y eine Zahl von 1 bis 10,
z eine Zahl von 1 bis 25,
m eine Zahl von 1 bis 2 und
n eine Zahl von 1 bis 2, wobei die Summe aus m + n höchstens 3 ist,
A eine der Gruppen entsprechend den nachstehenden Formeln 2 bis 10:

(2) 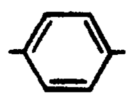

(3) 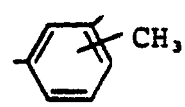

(4) 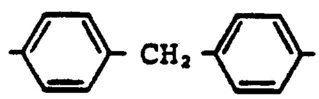

(5) 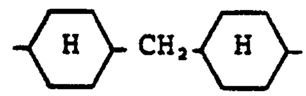

(6) 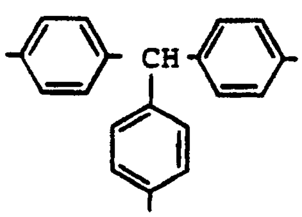

(7) $-(CH_2)_6-$

(8) 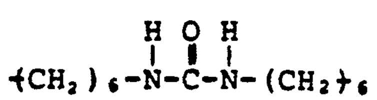

(9) 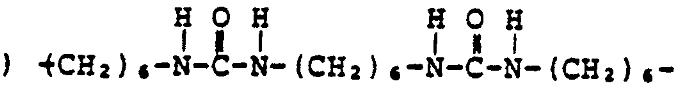

(10) 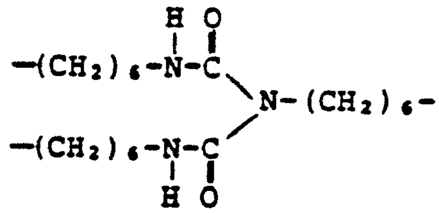

R eine Alkylengruppe mit 2 bis 6 C-Atomen, die Chlormethylethylengruppe oder eine Gruppe der Formel $-CH(CH_2OCH_2CH_2-C_4F_9$ bis $C_{20}F_{41})-CH_2-$, wobei R auch mehrere dieser Bedeutungen haben kann, wenn z größer als 1 ist, und

B das Wasserstoffatom, eine Alkylgruppe mit 1 bis 4 C-Atomen oder eine Gruppe entsprechend der nachstehenden Formel 11

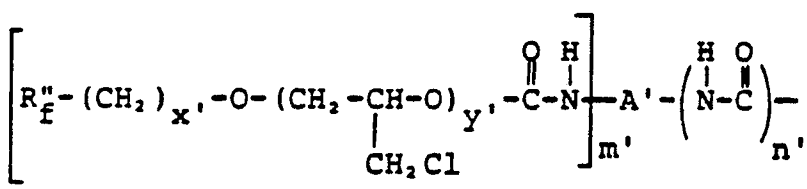 (11)

worin $R_f''$, x', y', m', n' und A' eine der Bedeutungen von $R_f$, x, y, m, n und A haben.

2. Urethane nach Anspruch 1, wobei bedeuten:

$R_f$ eine Perfluoralkylgruppe mit 6 bis 16 C-Atomen,
x 2,
y 1 bis 5,
z 1 bis 20,
m 1 bis 2,
n 1 bis 2, wobei die Summe m + n höchstens 3 ist,
A eine Toluylengruppe oder eine der drei Gruppen entsprechend den Formeln 8 bis 10,
R eine Ethylen-, Propylen-, Butylen- oder eine Chlormethylethylengruppe, wobei R auch mehrere dieser Bedeutungen haben kann, wenn z größer als 1 ist, und
B H, eine Alkylgruppe mit 1 bis 4 C-Atomen oder eine Gruppe entsprechend der Formel 11, wobei A' eine Toluylengruppe oder eine der drei Gruppen entsprechend den Formeln 8 bis 10 ist.

3. Urethane nach Anspruch 1, wobei bedeuten:

$R_f$ eine Perfluoralkylgruppe mit 6 bis 16 C-Atomen,
x 2,
y 1 bis 5,
z 1 bis 20,
m 1 bis 2,
n 1 bis 2, wobei die Summe m + n höchstens 3 ist,
A eine Toluylengruppe oder eine der drei Gruppen entsprechend den Formeln 8 bis 10,
R eine Ethylen-, Propylen-, Butylen- oder eine Chlormethylethylengruppe und im Fall von z größer als 1 auch eine Ethylen- und Propylengruppe, und
B H, eine Alkylgruppe mit 1 bis 4 C-Atomen oder eine Gruppe entsprechend Formel 11, wobei A' eine Toluylengruppe oder eine der drei Gruppen entsprechend den Formeln 8 bis 10 ist, $R_f''$ eine Perfluoralkylgruppe mit 6 bis 16 C-Atomen, x' 2, y' 1 bis 5, m' 1 bis 2 und n' 1 bis 2, wobei die Summe m' + n' höchstens 3 ist.

4. Verfahren zur Herstellung der Urethane nach Anspruch 1, gekennzeichnet durch Reaktion eines fluorhaltigen Alkohols der Formel

$R_f(CH_2)_xOH$,

worin $R_f$ und x die obengenannte Bedeutung haben, mit Epichlorhydrin zum Perfluorhydroalkanol-Epichlorhydrin-Addukt beziehungsweise Perfluorsulfonamidoalkanol-Epichlorhydrin-Addukt der Formel

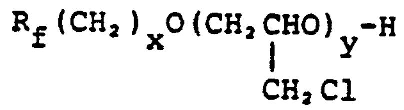

(Addukt 1)

worin $R_f$, x und y die obengenannte Bedeutung haben,
durch Reaktion des Adduktes 1 mit einem Di- oder Triisocyanat entsprechend einer der Gruppen gemäß Formeln 2 bis 10 zum Perfluoralkyl-Epichlorhydrin-Isocyanat-Addukt der Formel

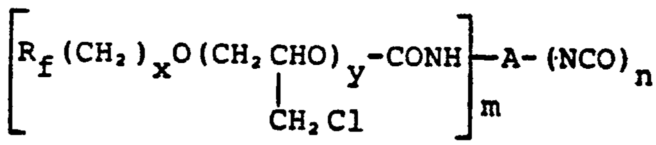

Addukt 2)

worin $R_f$, x, y, m und n die obengenannte Bedeutung haben,
und durch Reaktion des Adduktes 2 mit einem Dialkohol oder einem einseitig veretherten Dialkohol der nachstehenden Formeln

$H(OR)_zOH$ beziehungsweise $H(OR)_zOB$

worin R und z die obengenannte Bedeutung haben und B das Wasserstoffatom oder eine Alkylgruppe mit 1 bis 4 C-Atomen ist,
zu den angestrebten Perfluoralkyl- und Epichlorhydringruppen und Dialkoholreste enthaltenden Urethanen der beiden nachstehenden Formeln 12 und 13

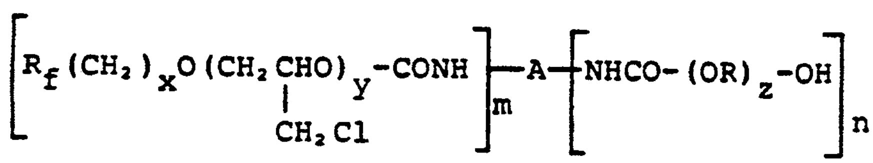

(12)

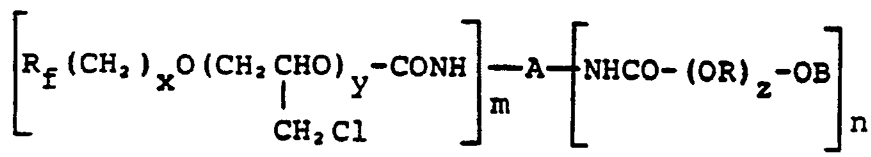

(13),

worin $R_f$, x, y, z, m, n, A und B die obengenannte Bedeutung haben,
und durch Reaktion eines Urethans der Formel 12 mit einer eine oder mehrere freie Isocyanatgruppen enthaltenden Isocyanatverbindung entsprechend der Gruppe gemäß Formel 11 zu den angestrebten Perfluoralkyl-und Epichlorhydringruppen und Dialkoholreste enthaltenden Urethanen der nachstehenden Formel 14

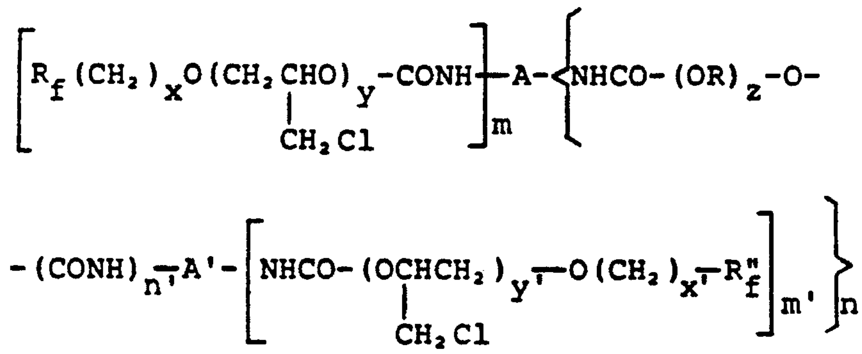 (14)

worin $R_f$, $R_f''$, x, x', y, y', z, m, m', n, n', A, A' und R die obengenannte Bedeutung haben.

5. Verwendung der Urethane nach Anspruch 1 zur Oleophob-und Hydrophob-Ausrüstung von Textilien.

**Patentansprüche für folgenden Vertragsstaat : AT**

1. Verfahren zur Herstellung von Perfluoralkylgruppen, von Epichlorhydrinabgeleiteten Gruppen und Dialkoholreste enthaltende Urethane der nachstehenden allgemeinen Formel 1

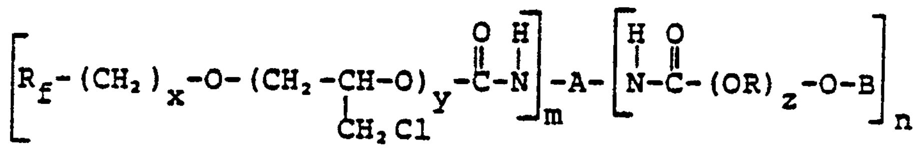 (1),

worin bedeuten:

- $R_f$ eine Perfluoralkylgruppe mit 4 bis 20 C-Atomen oder eine $R_f' SO_2NR_1$-Gruppe, in der $R_f'$ eine der Bedeutungen von $R_f$ hat und $R_1$ H oder eine Alkylgruppe mit 1 bis 4 C-Atomen ist,
- x eine ganze Zahl von 1 bis 4,
- y eine Zahl von 1 bis 10,
- z eine Zahl von 1 bis 25,
- m eine Zahl von 1 bis 2 und
- n eine Zahl von 1 bis 2, wobei die Summe aus m + n höchstens 3 ist,
- A eine der Gruppen entsprechend den nachstehenden Formeln 2 bis 10:

(2) 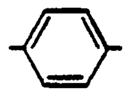

(3) 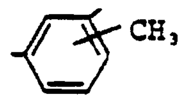

(4) 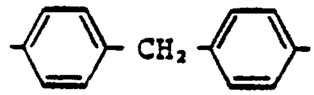

(5) 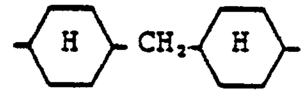

(6) 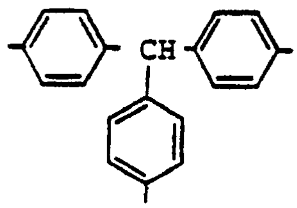

(7) $-(CH_2)_6-$

(8) 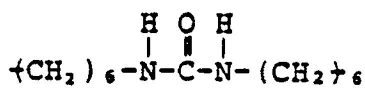

(9) 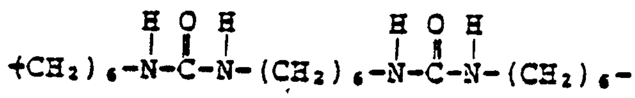

(10) 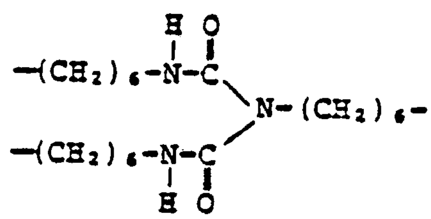

R eine Alkylengruppe mit 2 bis 6 C-Atomen, die Chlormethylethylengruppe oder eine Gruppe der Formel $-CH(CH_2OCH_2CH_2-C_4F_9$ bis $C_{20}F_{41})-CH_2-$, wobei R auch mehrere dieser Bedeutungen haben kann, wenn z größer als 1 ist, und

B das Wasserstoffatom, eine Alkylgruppe mit 1 bis 4 C-Atomen oder eine Gruppe entsprechend der nachstehenden Formel 11

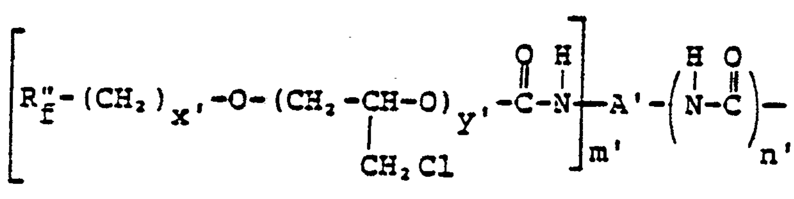 (11)

worin $R_f''$, x', y', m', n' und A' eine der Bedeutungen von $R_f$, x, y, m, n und A haben, gekennzeichnet

durch Reaktion eines fluorhaltigen Alkohols der Formel

$R_f(CH_2)_xOH$,

worin $R_f$ und x die obengenannte Bedeutung haben, mit Epichlorhydrin zum Perfluorhydroalkanol-Epichlorhydrin-Addukt beziehungsweise Perfluorsulfonamidoalkanol-Epichlorhydrin-Addukt der Formel

$$R_f(CH_2)_xO(CH_2\underset{\underset{CH_2Cl}{|}}{C}HO)_y-H \qquad \text{(Addukt 1)}$$

worin $R_f$, x und y die obengenannte Bedeutung haben,
durch Reaktion des Adduktes 1 mit einem Di- oder Triisocyanat entsprechend einer der Gruppen gemäß Formeln 2 bis 10 zum Perfluoralkyl-Epichlorhydrin-Isocyanat-Addukt der Formel

$$\left[R_f(CH_2)_xO(CH_2\underset{\underset{CH_2Cl}{|}}{C}HO)_y-CONH\right]_m-A-(NCO)_n \qquad \text{(Addukt 2)}$$

worin $R_f$, x, y, m und n die obengenannte Bedeutung haben,
und durch Reaktion des Adduktes 2 mit einem Dialkohol oder einem einseitig veretherten Dialkohol der nachstehenden Formeln

$H(OR)_zOH$ beziehungsweise $H(OR)_zOB$

worin R und z die obengenannte Bedeutung haben und B das Wasserstoffatom oder eine Alkylgruppe mit 1 bis 4 C-Atomen ist,
zu den angestrebten Perfluoralkyl- und Epichlorhydringruppen und Dialkoholreste enthaltenden Urethanen der beiden nachstehenden Formeln 12 und 13

$$\left[R_f(CH_2)_xO(CH_2\underset{\underset{CH_2Cl}{|}}{C}HO)_y-CONH\right]_m-A-\left[NHCO-(OR)_z-OH\right]_n \qquad (12)$$

$$\left[R_f(CH_2)_xO(CH_2\underset{\underset{CH_2Cl}{|}}{C}HO)_y-CONH\right]_m-A-\left[NHCO-(OR)_z-OB\right]_n \qquad (13),$$

worin $R_f$, x, y, z, m, n, A und B die obengenannte Bedeutung haben,
und durch Reaktion eines Urethans der Formel 12 mit einer eine oder mehrere freie Isocyanatgruppen enthaltenden Isocyanatverbindung entsprechend der Gruppe gemäß Formel 11 zu den angestrebten Perfluoralkyl-und Epichlorhydringruppen und Dialkoholreste enthaltenden Urethanen der nachstehenden Formel 14

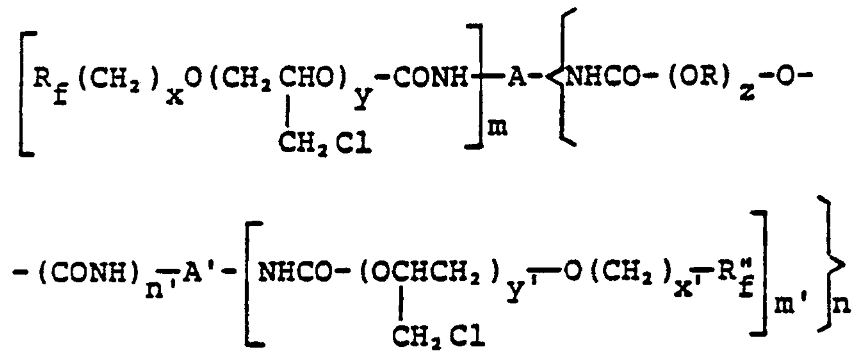 (14)

worin $R_f$, $R_f''$, x, x', y, y', z, m, m', n, n', A, A' und R die obengenannte Bedeutung haben.

2. Verwendung der nach Anspruch 1 hergestellten Urethane zur Oleophob- und Hydrophob-Ausrüstung von Textilien.

**Patentanspruch für folgenden Vertragsstaat : ES**

1. Verfahren zur Herstellung von Perfluoralkylgruppen, von Epichlorhydrinabgeleiteten Gruppen und Dialkoholreste enthaltende Urethane der nachstehenden allgemeinen Formel 1

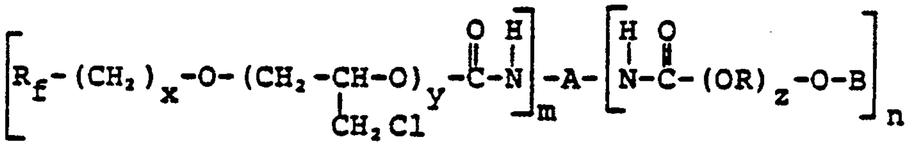 (1) ,

worin bedeuten:

- $R_f$ eine Perfluoralkylgruppe mit 4 bis 20 C-Atomen oder eine $R_f'$ $SO_2NR_1$-Gruppe, in der $R_f'$ eine der Bedeutungen von $R_f$ hat und $R_1$ H oder eine Alkylgruppe mit 1 bis 4 C-Atomen ist,
- x eine ganze Zahl von 1 bis 4,
- y eine Zahl von 1 bis 10,
- z eine Zahl von 1 bis 25,
- m eine Zahl von 1 bis 2 und
- n eine Zahl von 1 bis 2, wobei die Summe aus m + n höchstens 3 ist,
- A eine der Gruppen entsprechend den nachstehenden Formeln 2 bis 10:

(2) 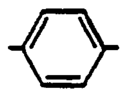

(3) 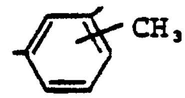

(4) 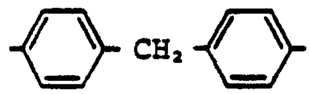

(5) 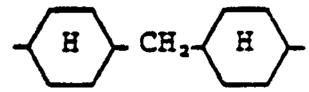

(6) 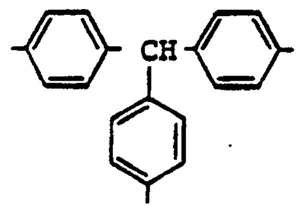

(7) $-(CH_2)_6-$

(8) 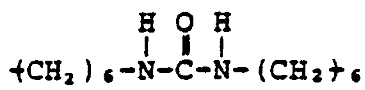

(9) 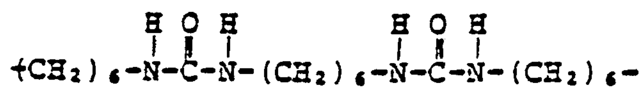

(10) 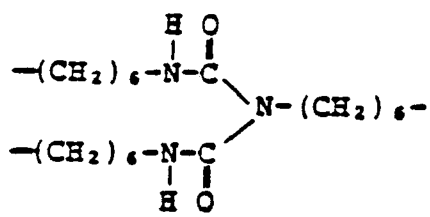

R eine Alkylengruppe mit 2 bis 6 C-Atomen, die Chlormethylethylengruppe oder eine Gruppe der Formel -$CH(CH_2OCH_2CH_2$-$C_4F_9$ bis $C_{20}F_{41})$-$CH_2$-, wobei R auch mehrere dieser Bedeutungen haben kann, wenn z größer als 1 ist, und

B das Wasserstoffatom, eine Alkylgruppe mit 1 bis 4 C-Atomen oder eine Gruppe entsprechend der nachstehenden Formel 11

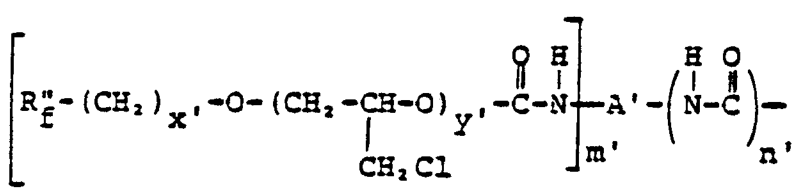 (11)

worin $R_f''$, x′, y′, m′, n′ und A′ eine der Bedeutungen von $R_f$, x, y, m, n und A haben, gekennzeichnet

durch Reaktion eines fluorhaltigen Alkohols der Formel

$R_f(CH_2)_xOH$,

worin $R_f$ und x die obengenannte Bedeutung haben, mit Epichlorhydrin zum Perfluorhydroalkanol-Epichlorhydrin-Addukt beziehungsweise Perfluorsulfonamidoalkanol-Epichlorhydrin-Addukt der Formel

$$R_f(CH_2)_xO(CH_2\underset{\substack{|\\CH_2Cl}}{C}HO)_y\text{-}H \qquad \text{(Addukt 1)}$$

worin $R_f$, x und y die obengenannte Bedeutung haben, durch Reaktion des Adduktes 1 mit einem Di- oder Triisocyanat entsprechend einer der Gruppen gemäß Formeln 2 bis 10 zum Perfluoralkyl-Epichlorhydrin-Isocyanat-Addukt der Formel

$$\left[R_f(CH_2)_xO(CH_2\underset{\substack{|\\CH_2Cl}}{C}HO)_y\text{-}CONH\right]_m\text{-}A\text{-}(NCO)_n \qquad \text{(Addukt 2)}$$

worin $R_f$, x, y, m und n die obengenannte Bedeutung haben, und durch Reaktion des Adduktes 2 mit einem Dialkohol oder einem einseitig veretherten Dialkohol der nachstehenden Formeln

$H(OR)_zOH$ beziehungsweise $H(OR)_zOB$

worin R und z die obengenannte Bedeutung haben und B das Wasserstoffatom oder eine Alkylgruppe mit 1 bis 4 C-Atomen ist, zu den angestrebten Perfluoralkyl- und Epichlorhydringruppen und Dialkoholreste enthaltenden Urethanen der beiden nachstehenden Formeln 12 und 13

$$\left[R_f(CH_2)_xO(CH_2\underset{\substack{|\\CH_2Cl}}{C}HO)_y\text{-}CONH\right]_m\text{-}A\text{-}\left[NHCO\text{-}(OR)_z\text{-}OH\right]_n \qquad (12)$$

$$\left[R_f(CH_2)_xO(CH_2\underset{\substack{|\\CH_2Cl}}{C}HO)_y\text{-}CONH\right]_m\text{-}A\text{-}\left[NHCO\text{-}(OR)_z\text{-}OB\right]_n \qquad (13),$$

worin $R_f$, x, y, z, m, n, A und B die obengenannte Bedeutung haben, und durch Reaktion eines Urethans der Formel 12 mit einer eine oder mehrere freie Isocyanatgruppen enthaltenden Isocyanatverbindung entsprechend der Gruppe gemäß Formel 11 zu den angestrebten Perfluoralkyl-und Epichlorhydringruppen und Dialkoholreste enthaltenden Urethanen der nachstehenden Formel 14

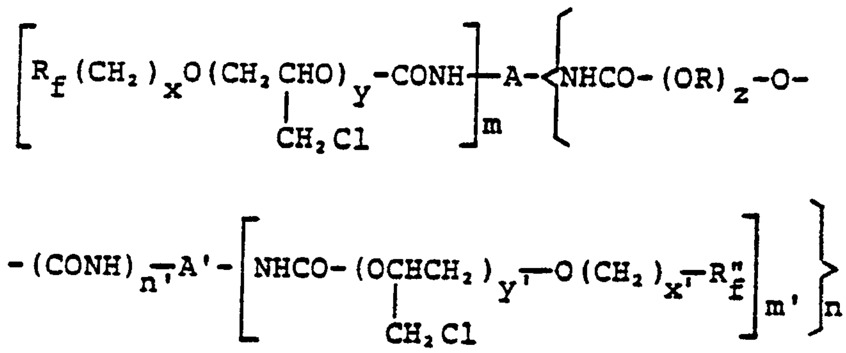

(14)

worin $R_f$, $R_f''$, x, x', y, y', z, m, m', n, n', A, A' und R die obengenannte Bedeutung haben.

## Claims

### Claims for the following Contracting States : BE, CH, DE, FR, GB, IT, LI, NL

**1.** Urethanes containing perfluoroalkyl groups, groups derived from epichlorohydrin and dialcohol radicals of the following formula 1

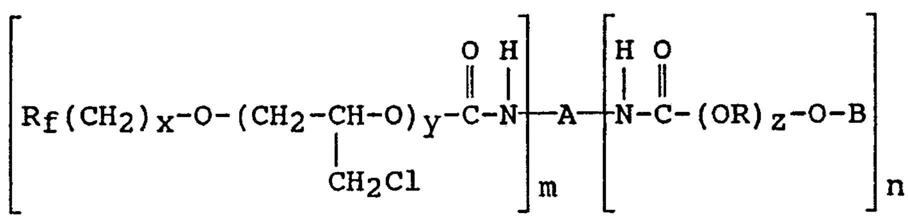

(1)

in which

- $R_f$ denotes a perfluoroalkyl group having 4 to 20 carbon atoms, or an $R'_fSO_2NR_1$ group in which $R'_f$ has one of the meanings of $R_f$, and $R_1$ is H or an alkyl group having 1 to 4 carbon atoms,
- x is a whole number from 1 to 4,
- y is a number from 1 to 10,
- z is a number from 1 to 25,
- m is a number from 1 to 2 and
- n is a number from 1 to 2, the sum of m + n being at most 3,
- A denotes one of the groups conforming to the following formulae 2 to 10:

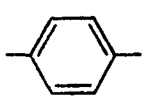

(2)

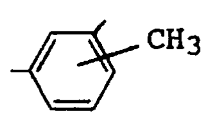

(3)

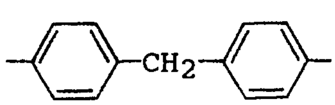

(4)

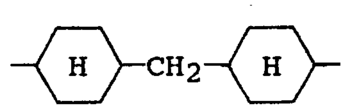

(5)

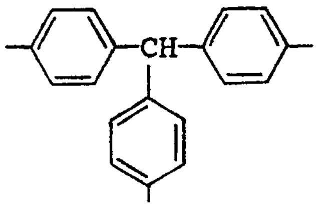 (6)

$-(CH_2)_6-$ (7)

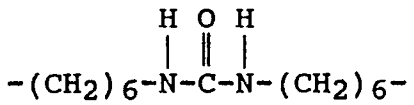 (8)

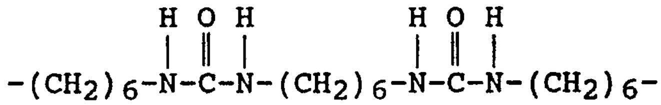 (9)

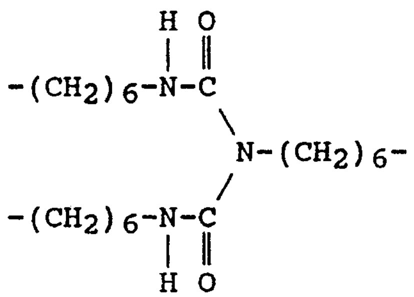 (10)

R denotes an alkylene group having 2 to 6 carbon atoms, the chloromethylethylene group or a group of the formula $-CH(CH_2OCH_2CH_2-C_4F_9$ to $C_{20}F_{41})-CH_2-$, where R can also have more than one of these meanings if z is greater than 1, and

B denotes the hydrogen atom, an alkyl group having 1 to 4 carbon atoms or a group conforming to the following formula 11

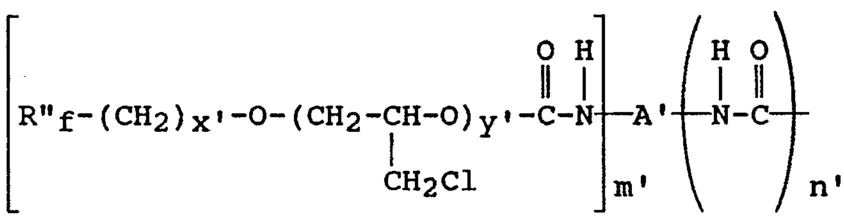 (11)

in which $R''_f$, x', y', m', n', and A' have one of the meanings of $R_f$, x, y, m , n, and A.

**2.** The urethane as claimed in claim 1, wherein

- $R_f$ denotes a perfluoroalkyl group having 6 to 16 carbon atoms,
- x denotes 2,
- y denotes 1 to 5,
- z denotes 1 to 20,
- m denotes 1 to 2,
- n denotes 1 to 2, the sum of m + n being at most 3,
- A denotes a toluylene group or one of the three groups conforming to the formulae 8 to 10,
- R denotes an ehtylene, propylene, butylene or a chloromethylethylene group, where R can also have more than one of these meanings if z is greater than 1, and

B denotes H, an alkyl group having 1 to 4 carbon atoms or a group conforming to the formula 11, where A' is a toluylene group or one of the three groups conforming to the formulae 8 to 10.

**3.** The urethane as claimed in claim 1, wherein

- $R_f$ denotes a perfluoroalkyl group having 6 to 16 carbon atoms,
- x denotes 2,
- y denotes 1 to 5,
- z denotes 1 to 20,
- m denotes 1 to 2,
- n denotes 1 to 2, the sum of m + n being at most 3,
- A denotes a toluylene group or one of the three groups conforming to the formulae 8 to 10,
- R denotes an ethylene, propylene, butylene or a chloromethylethylene group and in the case of z being greater than 1 also an ethylene and propylene group, and
- B denotes H, an alkyl group having 1 to 4 carbon atoms or a group conforming to the formula 11, where A' is a toluylene group or one of the three groups conforming to the formulae 8 to 10, $R''_f$ is a perfluoroalkyl group having 6 to 16 carbon atoms, x' is 2, y' is 1 to 5, m' is 1 to 2, and n' is 1 to 2, the sum of m' + n' being at most 3.

**4.** A process for preparing the urethane as claimed in claim 1, by reacting a fluorine-containing alcohol of the formula

$R_f(CH_2)_xOH$,

in which $R_f$ and x have the above-mentioned meaning with epichlorohydrin to give the perfluorohydroalkanol-epichlorohydrin adduct or perfluorosulfonamidoalkanol-epichlorohydrin adduct of the formula

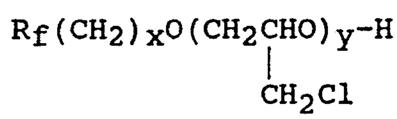
(adduct 1)

in which $R_f$, x, and y have the above-mentioned meaning,
by reacting adduct 1 with a di- or triisocyanate conforming to one of the groups of the formulae 2 to 10 to give the perfluoroalkyl-epichlorohydrin-isocyanate adduct of the formula

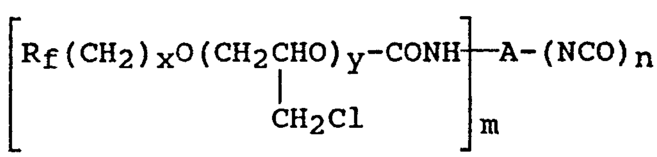
(adduct 2)

in which $R_f$, x, y, m, and n have the above-mentioned meaning,
and by reacting adduct 2 with a dialcohol or a monoetherified dialcohol of the following formulae

$H(OR)_zOH$ or $H(OR)_zOB$

in which R and z have the above-mentioned meanings and B is the hydrogen atom or an alkyl group having 1 to 4 carbon atoms,
to give the desired urethanes which contain perfluoroalkyl and epichlorohydrin groups and dialcohol radicals and have the two following formulae 12 and 13

$$\left[R_f(CH_2)_xO(CH_2\underset{\underset{CH_2Cl}{|}}{C}HO)_y-CONH\right]_m-A-\left[NHCO-(OR)_z-OH\right]_n \qquad (12)$$

$$\left[R_f(CH_2)_xO(CH_2\underset{\underset{CH_2Cl}{|}}{C}HO)_y-CONH\right]_m-A-\left[NHCO-(OR)_z-OB\right]_n \qquad (13)$$

in which $R_f$, x, y, z, m, n, A, and B have the above-mentioned meaning, and by reacting a urethane of the formula 12 with an isocyanate compound which contains one or more free isocyanate groups and conforms to the group of the formula 11 to give the desired urethanes which contain perfluoroalkyl and epichlorohydrin groups and dialcohol radicals and have the following formula 14

$$\left[R_f(CH_2)_xO(CH_2\underset{\underset{CH_2Cl}{|}}{C}HO)_y-CONH\right]_m-A-\Bigg\{NHCO-(OR)_z-O-$$

$$-(CONH)_{n'}-A'-\left[NHCO-(O\underset{\underset{CH_2Cl}{|}}{C}HCH_2)_{y'}-O(CH_2)_{x'}-R''_f\right]_{m'}\Bigg\}_n \qquad (14)$$

in which $R_f$, $R''_f$, x, x', y, y', z, m, m', n, n', A, A', and R have the above-mentioned meaning.

5. The use of the urethanes as claimed in claim 1 for the oleophobic and hydrophobic finishing of textiles.

**Claims for the following Contracting State : AT**

1. A process for preparing urethanes containing perfluoroalkyl groups, groups derived from epichlorohydrin and dialcohol radicals of the following formula 1

$$\left[R_f(CH_2)_x-O-(CH_2-\underset{\underset{CH_2Cl}{|}}{C}H-O)_y-\overset{\overset{O}{\|}}{C}-\overset{\overset{H}{|}}{N}\right]_m-A-\left[\overset{\overset{H}{|}}{N}-\overset{\overset{O}{\|}}{C}-(OR)_z-O-B\right]_n \qquad (1)$$

in which

$R_f$ denotes a perfluoroalkyl group having 4 to 20 carbon atoms, or an $R'_fSO_2NR_1$ group in which $R'_f$ has one of the meanings of $R_f$, and $R_1$ is H or an alkyl group having 1 to 4 carbon atoms,

x is a whole number from 1 to 4,
y is a number from 1 to 10,
z is a number from 1 to 25,
m is a number from 1 to 2 and
n is a number from 1 to 2, the sum of m + n being at most 3,
A denotes one of the groups conforming to the following formulae 2 to 10:

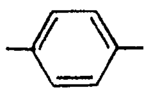 (2)

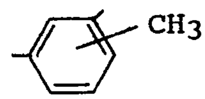 (3)

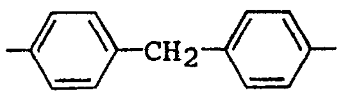 (4)

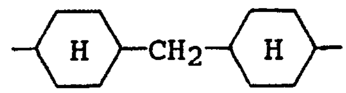 (5)

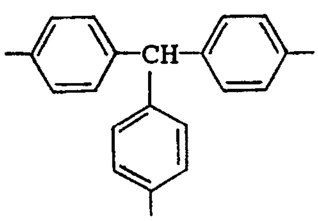 (6)

$-(CH_2)_6-$ (7)

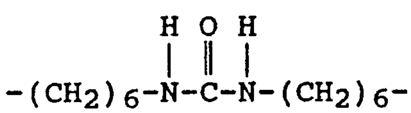 (8)

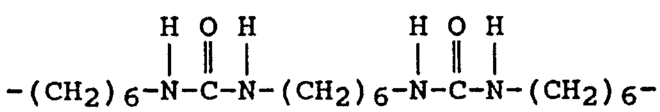 (9)

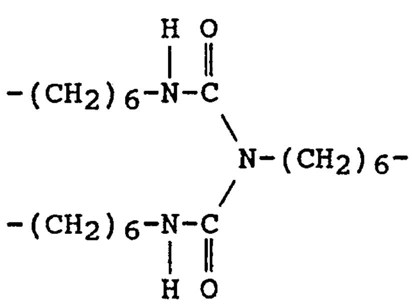 (10)

R denotes an alkylene group having 2 to 6 carbon atoms, the chloromethylethylene group or a group of the formula $-CH(CH_2OCH_2CH_2-C_4F_9$ to $C_{20}F_{41})-CH_2-$, where R can also have more than one of these meanings if z is greater than 1, and
B denotes the hydrogen atom, an alkyl group having 1 to 4 carbon atoms or a group conforming to the following formula 11

$$\left[R''_f-(CH_2)_{x'}-O-(CH_2-\underset{\displaystyle CH_2Cl}{\underset{|}{CH}}-O)_{y'}-\overset{O}{\overset{\|}{C}}-\overset{H}{\overset{|}{N}}\right]_{m'}-A'-\left(-\overset{H}{\overset{|}{N}}-\overset{O}{\overset{\|}{C}}-\right)_{n'} \qquad (11)$$

in which $R''_f$, x', y', m', n', and A' have one of the meanings of $R_f$, x, y, m , n, and A, characterised by reacting a fluorine-containing alcohol of the formula

$R_f(CH_2)_xOH$,

in which $R_f$ and x have the above-mentioned meaning with epichlorohydrin to give the perfluorohydroalkanol-epichlorohydrin adduct or perfluorosulfonamidoalkanol-epichlorohydrin adduct of the formula

$$R_f(CH_2)_xO(CH_2\underset{\displaystyle CH_2Cl}{\underset{|}{C}}HO)_y-H \qquad \text{(adduct 1)}$$

in which $R_f$, x, and y have the above-mentioned meaning,
by reacting adduct 1 with a di- or triisocyanate conforming to one of the groups of the formulae 2 to 10 to give the perfluoroalkyl-epichlorohydrin-isocyanate adduct of the formula

$$\left[R_f(CH_2)_xO(CH_2\underset{\displaystyle CH_2Cl}{\underset{|}{C}}HO)_y-CONH\right]_m-A-(NCO)_n \qquad \text{(adduct 2)}$$

in which $R_f$, x, y, m, and n have the above-mentioned meaning,
and by reacting adduct 2 with a dialcohol or a monoetherified dialcohol of the following formulae

$H(OR)_zOH$ or $H(OR)_zOB$

in which R and z have the above-mentioned meanings and B is the hydrogen atom or an alkyl group having 1 to 4 carbon atoms,
to give the desired urethanes which contain perfluoroalkyl and epichlorohydrin groups and dialcohol radicals and have the two following formulae 12 and 13

$$\left[R_f(CH_2)_xO(CH_2\underset{\displaystyle CH_2Cl}{\underset{|}{C}}HO)_y-CONH\right]_m-A-\left[NHCO-(OR)_z-OH\right]_n \qquad (12)$$

$$\left[R_f(CH_2)_xO(CH_2\underset{\displaystyle CH_2Cl}{\underset{|}{C}}HO)_y-CONH\right]_m-A-\left[NHCO-(OR)_z-OB\right]_n \qquad (13)$$

in which $R_f$, x, y, z, m, n, A, and B have the above-mentioned meaning, and by reacting a urethane of the formula 12 with an isocyanate compound which contains one or more free isocyanate groups and conforms to the group of the formula 11 to give the desired urethanes which contain perfluoroalkyl and epichlorohydrin groups and dialcohol radicals and have the following formula 14

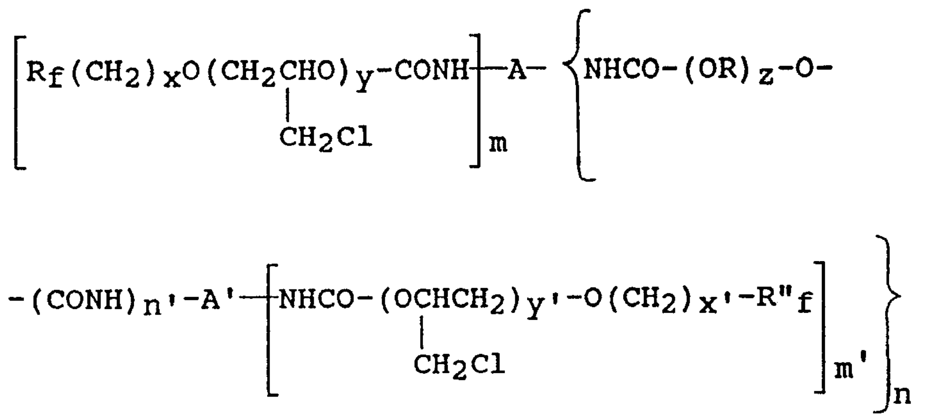

(14)

in which $R_f$, $R''_f$, x, x', y, y', z, m, m', n, n', A, A', and R have the above-mentioned meaning.

2. The use of the urethanes as made in claim 1 for the oleophobic and hydrophobic finishing of textiles.

**Claim for the following Contracting State : ES**

1. A process for preparing urethanes containing perfluoroalkyl groups, groups derived from epichlorohydrin and dialcohol radicals of the following formula 1

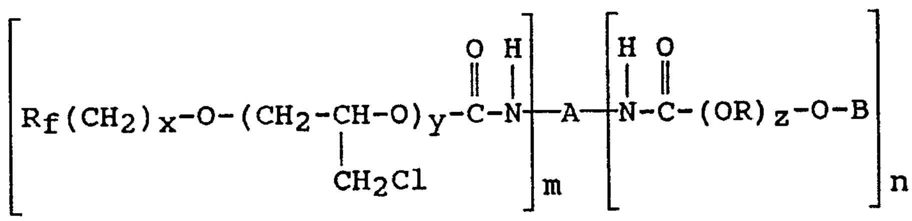

(1)

in which

| | |
|---|---|
| $R_f$ | denotes a perfluoroalkyl group having 4 to 20 carbon atoms, or an $R'_fSO_2NR_1$ group in which $R'_f$ has one of the meanings of $R_f$, and $R_1$ is H or an alkyl group having 1 to 4 carbon atoms, |
| x | is a whole number from 1 to 4, |
| y | is a number from 1 to 10, |
| z | is a number from 1 to 25, |
| m | is a number from 1 to 2 and |
| n | is a number from 1 to 2, the sum of m + n being at most 3, |
| A | denotes one of the groups conforming to the following formulae 2 to 10: |

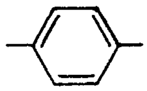 (2)

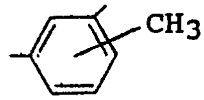 (3)

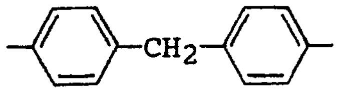 (4)

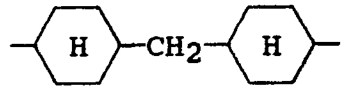 (5)

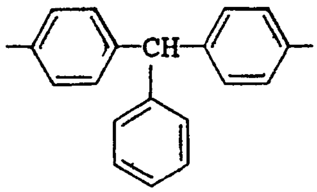 (6)

$-(CH_2)_6-$ (7)

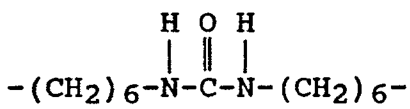 (8)

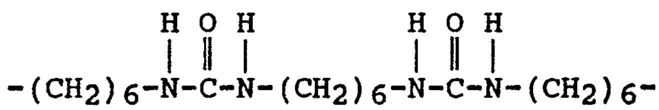 (9)

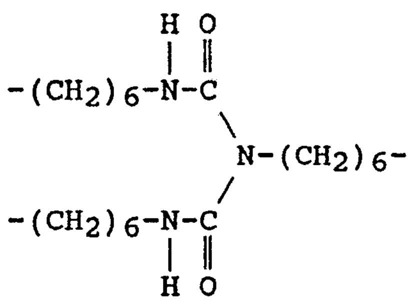 (10)

R denotes an alkylene group having 2 to 6 carbon atoms, the chloromethylethylene group or a group of the formula $-CH(CH_2OCH_2CH_2-C_4F_9$ to $C_{20}F_{41})-CH_2-$, where R can also have more than one of these meanings if z is greater than 1, and

B denotes the hydrogen atom, an alkyl group having 1 to 4 carbon atoms or a group conforming to the following formula 11

$$\left[R''_f-(CH_2)_{x'}-O-(CH_2-\underset{\displaystyle CH_2Cl}{\underset{|}{CH}}-O)_{y'}-\overset{\displaystyle O}{\overset{\|}{C}}-\overset{\displaystyle H}{\overset{|}{N}}\right]_{m'}A'\left(-\overset{\displaystyle H}{\overset{|}{N}}-\overset{\displaystyle O}{\overset{\|}{C}}-\right)_{n'} \qquad (11)$$

in which $R''_f$, x', y', m', n', and A' have one of the meanings of $R_f$, x, y, m , n, and A, characterised by reacting a fluorine-containing alcohol of the formula

$R_f(CH_2)_xOH$,

in which $R_f$ and x have the above-mentioned meaning with epichlorohydrin to give the perfluorohydroalkanol-epichlorohydrin adduct or perfluorosulfonamidoalkanol-epichlorohydrin adduct of the formula

$$R_f(CH_2)_xO(CH_2\underset{\displaystyle CH_2Cl}{\underset{|}{CH}}O)_y-H \qquad \text{(adduct 1)}$$

in which $R_f$, X, and y have the above-mentioned meaning,
by reacting adduct 1 with a di- or triisocyanate conforming to one of the groups of the formulae 2 to 10 to give the perfluoroalkyl-epichlorohydrin-isocyanate adduct of the formula

$$\left[R_f(CH_2)_xO(CH_2\underset{\displaystyle CH_2Cl}{\underset{|}{CH}}O)_y-CONH\right]_m A-(NCO)_n \qquad \text{(adduct 2)}$$

in which $R_f$, x, y, m, and n have the above-mentioned meaning,
and by reacting adduct 2 with a dialcohol or a monoetherified dialcohol of the following formulae

$H(OR)_zOH$ or $H(OR)_zOB$

in which R and z have the above-mentioned meanings and B is the hydrogen atom or an alkyl group having 1 to 4 carbon atoms,
to give the desired urethanes which contain perfluoroalkyl and epichlorohydrin groups and dialcohol radicals and have the two following formulae 12 and 13

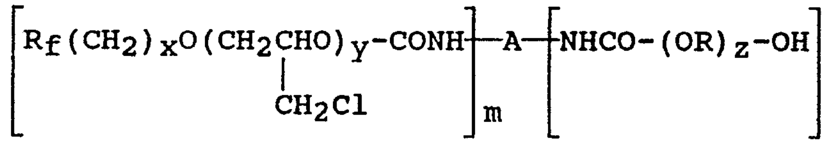 (12)

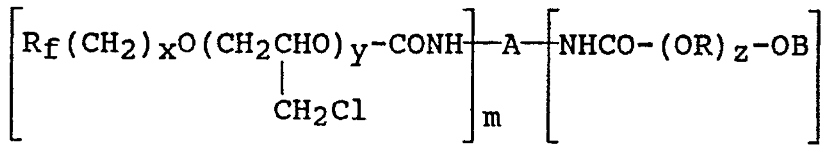 (13)

in which $R_f$, x, y, z, m, n, A, and B have the above-mentioned meaning, and by reacting a urethane of the formula 12 with an isocyanate compound which contains one or more free isocyanate groups and conforms to the group of the formula 11 to give the desired urethanes which contain perfluoroalkyl and epichlorohydrin groups and dialcohol radicals and have the following formula 14

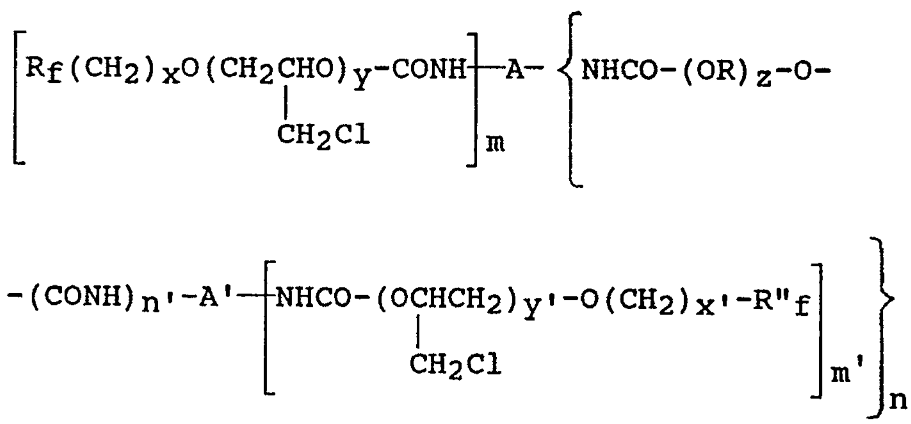 (14)

in which $R_f$, $R''_f$, x, x', y, y', z, m, m', n, n', A, A', and R have the above-mentioned meaning.

**Revendications**

**Revendications pour les Etats contractants suivants : BE, CH, DE, FR, GB, IT, LI, NL**

1. Uréthanes à groupes perfluoralkyliques et groupes d'épichlorhydrine et à radicaux de dialcools, de formule générale 1 ci-dessous :

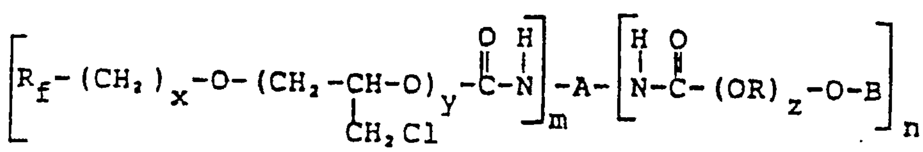 (1),

dans laquelle

| | |
|---|---|
| $R_f$ | désigne un groupe perfluoralkylique en $C_4$ à $C_{20}$, ou un groupe $R'_fSO_2NR_1$- dont R'f a la même définition que $R_f$ et $R_1$ est l'hydrogène ou un alkyle en $C_1$ à $C_4$, |
| x | est un entier de 1 à 4, |
| y | un nombre de 1 à 10, |
| z | un nombre de 1 à 25, |
| m et n | sont chacun un nombre de 1 à 2 et la somme m + n est au maximum égale à 3, |
| A | représente l'un des groupes de formules 2 à 10 suivants, qui sont des groupes sans |

radical isocyanate :

(2) 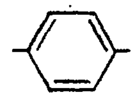

(3) 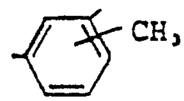

(4) 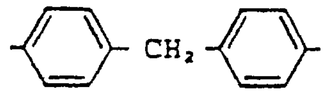

(5) 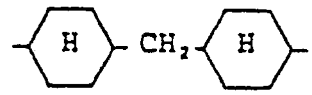

(6) 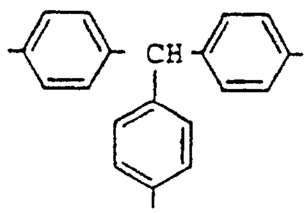

(7) $-(CH_2)_6-$

(8) 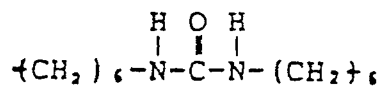

(9) 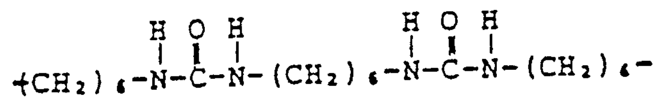

(10) 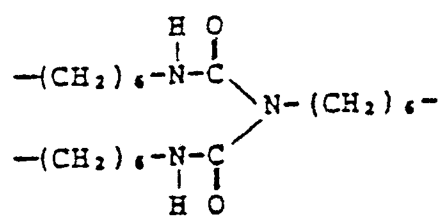

R est un alkylène en $C_2$ à $C_6$, ou bien le groupe chlorométhyléthylène ou un groupe $-CH(CH_2OCH_2CH_2R_F)-CH_2-$ ($R_F$ étant un radical de $C_4F_9$ à $C_{20}F_{41}$), R pouvant aussi représenter plusieurs de ces groupes à la fois si z est supérieur à 1, et

B est un atome d'hydrogène, un alkyle en $C_1$ à $C_4$ ou un groupe de formule 11 ci-dessous :

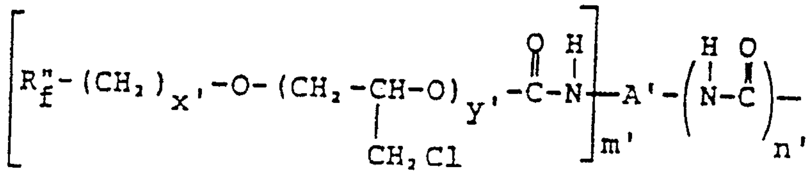 (11)

dans laquelle les divers symboles ont l'une des significations qui ont été indiquées pour $R_f$, x, y, m, n et A.

**2.** Uréthanes selon la revendication 1 dans lesquels :

$R_f$ est un groupe perfluoralkylique ayant de 6 à 16 atomes de carbone,
x est le nombre 2,
y un nombre de 1 à 5,
z un nombre de 1 à 20,
m un nombre de 1 à 2,
n un nombre de 1 à 2, la somme m + n étant au maximum égale à 3,
A est un groupe toluylène ou l'un des trois groupes de formules 8 à 10
R un groupe éthylène, propylène, butylène ou chlorométhyléthylène, ou pouvant aussi représenter plusieurs de ces groupes à la fois si z est supérieur à 1, et
B représente l'hydrogène, un alkyle en $C_1$ à $C_4$ ou un groupe de formule 11 dans lequel A' est un groupe toluylène ou l'un des trois groupes de formules 8 à 10.

**3.** Uréthanes selon la revendication 1 dans lesquels :
$R_f$ est un groupe perfluoralkylique ayant de 6 à 16 atomes de carbone,
x est le nombre 2,
y un nombre de 1 à 5,
z un nombre de 1 à 20,
m un nombre de 1 à 2,
n un nombre de 1 à 2, la somme m + n étant au maximum égale à 3,
A est un groupe toluylène ou l'un des trois groupes de formules 8 à 10
R un groupe éthylène, propylène, butylène ou chlorométhyléthylène, ou encore représente à la fois les groupes éthylène et propylène si z est supérieur à 1, et
B représente l'hydrogène, un alkyle en $C_1$ à $C_4$ ou un groupe de formule 11 dans lequel A' est un groupe toluylène ou l'un des trois groupes de formules 8 à 10, R"f un groupe perfluoralkylique ayant de 6 à 16 atomes de carbone, x' le nombre 2, y' un nombre de 1 à 5, m' un nombre de 1 à 2 et n' un nombre de 1 à 2, la somme m' + n' étant au maximum égale à 3.

**4.** Procédé de préparation des uréthanes selon la revendication 1, procédé caractérisé en ce que l'on fait réagir un alcool fluoré de formule :

$R_f(CH_2)_xOH$,

avec l'épichlorhydrine pour former le produit d'addition du perfluorhydroalcanol ou du perfluorosulfonamidoalcanol et de l'épichlorhydrine, de formule :

$$R_f(CH_2)_xO(CH_2\underset{\displaystyle CH_2Cl}{\underset{|}{C}}HO)_y-H \qquad \text{(produit d'addition 1)}$$

on fait réagir ce produit d'addition 1 avec un diisocyanate ou un triisocyanate correspondant à l'un des groupes 2 à 10 ci-dessus pour former le produit d'addition perfluoralkyl-épichlorhydrine-isocyanate, de formule

$$\left[R_f(CH_2)_xO(CH_2\underset{\displaystyle CH_2Cl}{\underset{|}{C}}HO)_y-CONH\right]_m-A-(NCO)_n \qquad \text{(produit d'addition 2)}$$

et on fait enfin réagir ce produit d'addition 2 avec un dialcool (diol) ou un monoéther de diol, de formule

$H(OR)_zOH$ ou $H(OR)_zOB$

B désignant l'hydrogène ou un alkyle en $C_1$ à $C_4$,
ce qui forme les uréthanes voulus à groupes perfluoralkyliques et d'épichlorhydrine et à radicaux de

dialcools, de formules 12 et 13 ci-dessous :

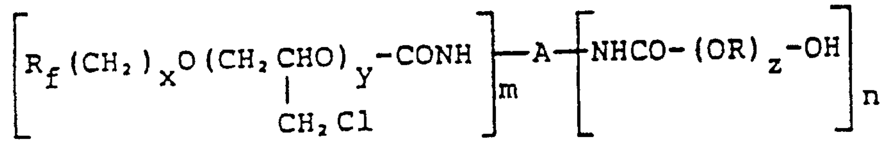

(12)

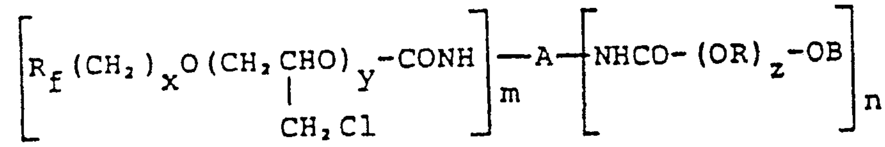

(13),

et par réaction d'un uréthane de formule 12 avec un isocyanate ayant un ou plusieurs groupes isocyanate libres, correspondant au groupe de formule 11, on forme les uréthanes voulus à groupes perfluoralkyliques et d'épichlorhydrine et à radicaux de dialcools, de formule 14

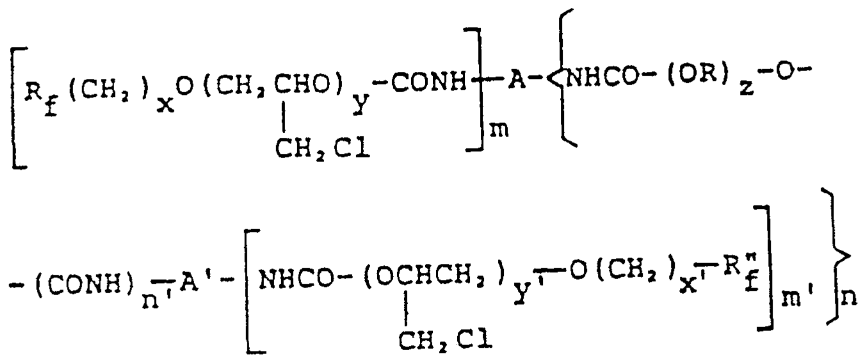

(14)

les divers symboles ayant les significations précédemment données.

**5.** L'emploi des uréthanes de la revendication 1 pour rendre des matières textiles oléphobes et hydrophobes.

**Revendications pour l'Etat contractant suivant : AT**

**1.** Procédé de préparation d'uréthanes à groupes perfluoralkyliques et groupes d'épichlorhydrine et à radicaux de dialcools, de formule générale 1 ci-dessous :

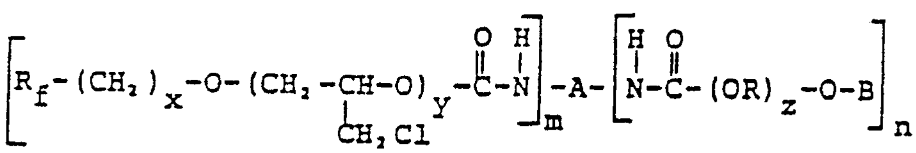

(1),

dans laquelle

| | |
|---|---|
| $R_f$ | désigne un groupe perfluoralkylique en $C_4$ à $C_{20}$, ou un groupe $R'_fSO_2NR_1$- dont R'f a la même définition que $R_f$ et $R_1$ est l'hydrogène ou un alkyle en $C_1$ à $C_4$, |
| x | est un entier de 1 à 4, |
| y | un nombre de 1 à 10, |
| z | un nombre de 1 à 25, |
| m et n | sont chacun un nombre de 1 à 2 et la somme m + n est au maximum égale à 3, |
| A | représente l'un des groupes 2 à 10 suivants, qui sont des groupes sans radical |

isocyanate :

(2) 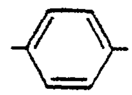

(3) 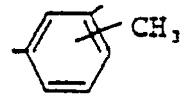

(4) 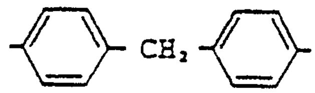

(5) 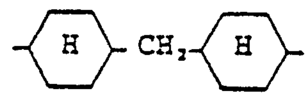

(6) 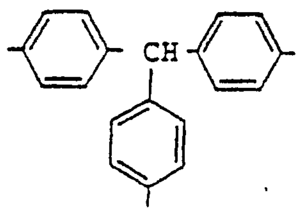

(7) $-(CH_2)_6-$

(8) 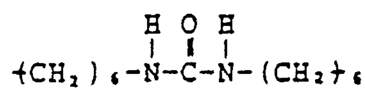

(9) 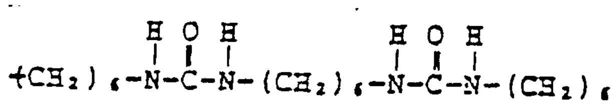

(10) 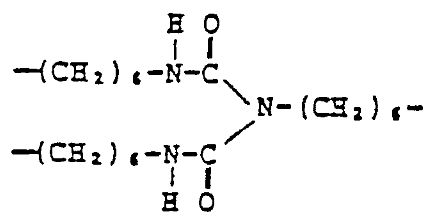

R est un alkylène en $C_2$ à $C_6$, ou bien le groupe chlorométhyléthylène ou un groupe $-CH(CH_2OCH_2CH_2RF)-CH_2-$ ($R_F$ étant un radical de $C_4F_9$ à $C_{20}F_{41}$), R pouvant aussi représenter plusieurs de ces groupes à la fois si z est supérieur à 1, et

B est un atome d'hydrogène, un alkyle en $C_1$ à $C_4$ ou un groupe de formule 11 ci-dessous :

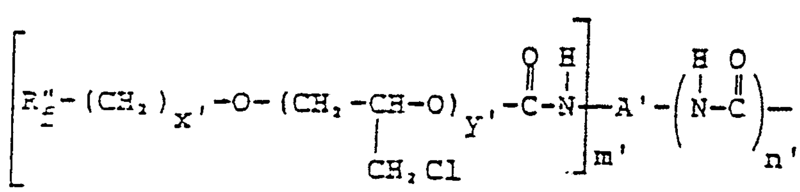 (11)

dans laquelle les divers symboles ont l'une des significations qui ont été indiquées pour $R_f$, x, y, m, n et A,

procédé caractérisé en ce qu'on fait réagir un alcool fluoré de formule :

$R_f(CH_2)_xOH$,

avec l'épichlorhydrine pour former le produit d'addition du perfluorhydroalcanol ou du perfluorosulfona-

midoalcanol et de l'épichlorhydrine, de formule :

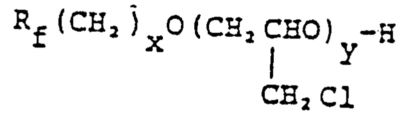 (produit d'addition 1)

on fait réagir ce produit d'addition 1 avec un diisocyanate ou un triisocyanate correspondant à l'un des groupes 2 à 10 ci-dessus pour former le produit d'addition perfluoralkylépichlorhydrine-isocyanate, de formule

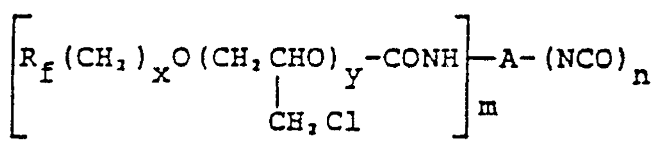 (produit d'addition 2)

et on fait enfin réagir ce produit d'addition 2 avec un dialcool (diol) ou un monoéther de diol, de formule

$H(OR)_zOH$ ou $H(OR)_zOB$

B désignant l'hydrogène ou un alkyle en $C_1$ à $C_4$,
ce qui forme les uréthanes voulus à groupes perfluoralkyliques et d'épichlorhydrine et à radicaux de dialcools, de formules 12 et 13 ci-dessous :

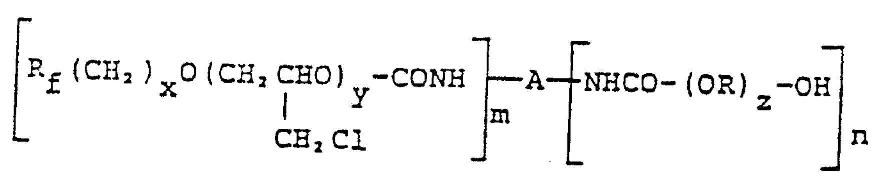 (12)

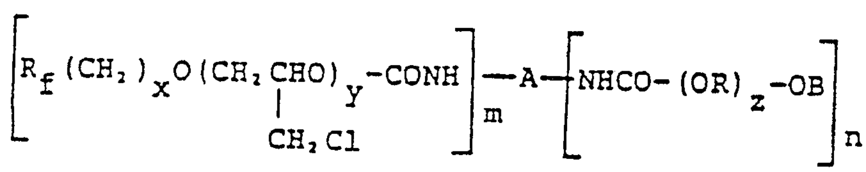 (13),

et par réaction d'un uréthane de formule 12 avec un isocyanate ayant un ou plusieurs groupes isocyanate libres, correspondant au groupe de formule 11, on forme les uréthanes voulus à groupes perfluoralkyliques et d'épichlorhydrine et à radicaux de dialcools, de formule 14

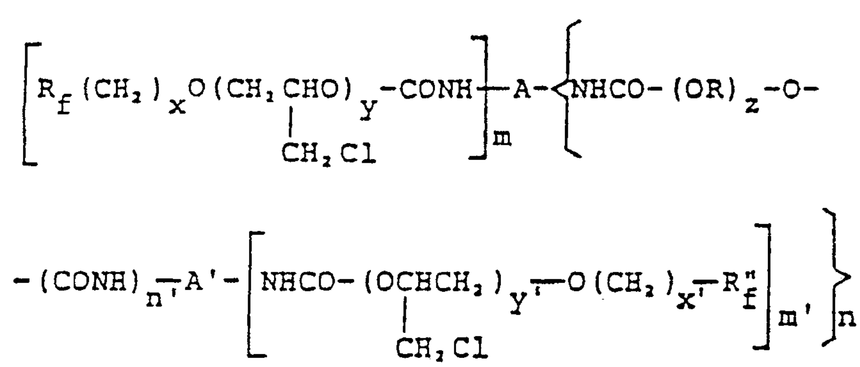 (14)

les divers symboles ayant les significations précédemment données.

2. L'emploi des uréthanes de la revendication 1 pour rendre des matières textiles oléphobes et hydrophobes.

**Revendication pour l'Etat contractant suivant : ES**

1. Procédé de préparation d'uréthanes à groupes perfluoralkyliques et groupes d'épichlorhydrine et à radicaux de dialcools, de formule générale 1 ci-dessous :

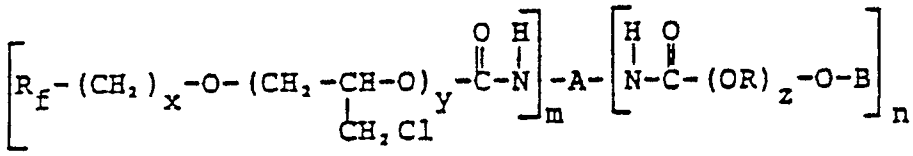 (1),

dans laquelle

$R_f$ désigne un groupe perfluoralkylique en $C_4$ à $C_{20}$, ou un groupe $R'_fSO_2NR_1$- dont R'f a la même définition que $R_f$ et $R_1$ est l'hydrogène ou un alkyle en $C_1$ à $C_4$,

x est un entier de 1 à 4,

y un nombre de 1 à 10,

z un nombre de 1 à 25,

m et n sont chacun un nombre de 1 à 2 et la somme m + n est au maximum égale à 3,

A représente l'un des groupes 2 à 10 suivants, qui sont des groupes sans radical isocyanate :

(2) 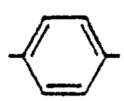

(3) 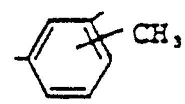

(4) 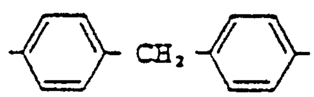

(5) 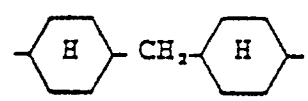

(6) 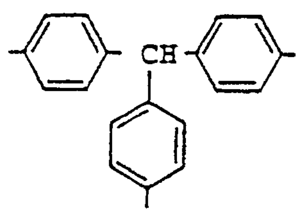

(7) $-(CH_2)_6-$

(8) 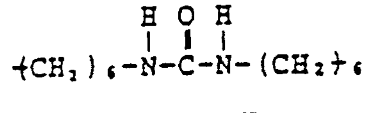

(9) 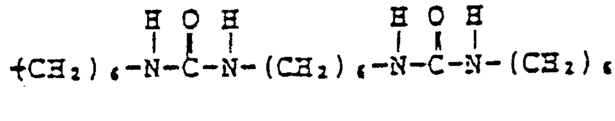

(10) 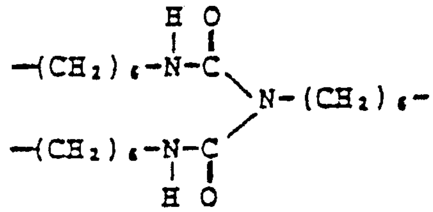

R est un alkylène en $C_2$ à $C_6$, ou bien le groupe chlorométhyléthylène ou un groupe $-CH(CH_2OCH_2CH_2RF)-CH_2-$ ($R_F$ étant un radical de $C_4F_9$ à $C_{20}F_{41}$), R pouvant aussi représenter plusieurs de ces groupes à la fois si z est supérieur à 1, et

B est un atome d'hydrogène, un alkyle en $C_1$ à $C_4$ ou un groupe de formule 11 ci-dessous :

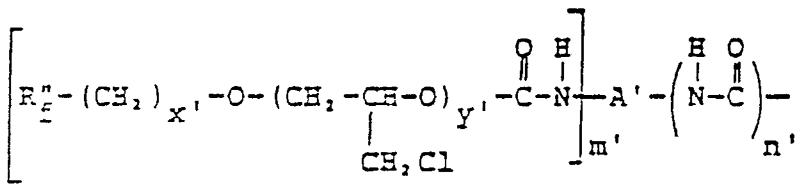 (11)

dans laquelle les divers symboles ont l'une des significations qui ont été indiquées pour $R_f$, x, y, m, n et A,

procédé caractérisé en ce qu'on fait réagir un alcool fluoré de formule :

$R_f(CH_2)_xOH$,

avec l'épichlorhydrine pour former le produit d'addition du perfluorhydroalcanol ou du perfluorosulfonamidoalcanol et de l'épichlorhydrine, de formule :

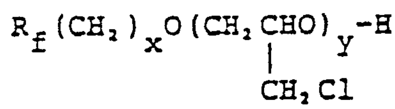 (produit d'addition 1)

on fait réagir ce produit d'addition 1 avec un diisocyanate ou un triisocyanate correspondant à l'un des groupes 2 à 10 ci-dessus pour former le produit d'addition perfluoralkylépichlorhydrine-isocyanate, de formule

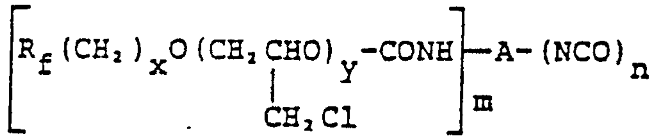 (produit d'addition 2)

et on fait enfin réagir ce produit d'addition 2 avec un dialcool (diol) ou un monoéther de diol, de formule

$H(OR)_zOH$ ou $H(OR)_zOB$

B désignant l'hydrogène ou un alkyle en $C_1$ à $C_4$,
ce qui forme les uréthanes voulus à groupes perfluoralkyliques et d'épichlorhydrine et à radicaux de dialcools, de formules 12 et 13 ci-dessous :

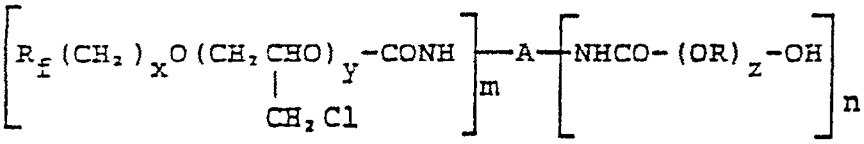 (12)

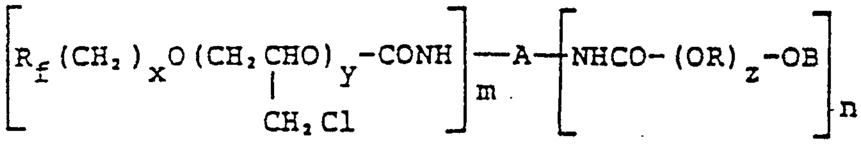 (13),

et par réaction d'un uréthane de formule 12 avec un isocyanate ayant un ou plusieurs groupes isocyanate libres, correspondant au groupe de formule 11, on forme les uréthanes voulus à groupes perfluoralkyliques et d'épichlorhydrine et à radicaux de dialcools, de formule 14

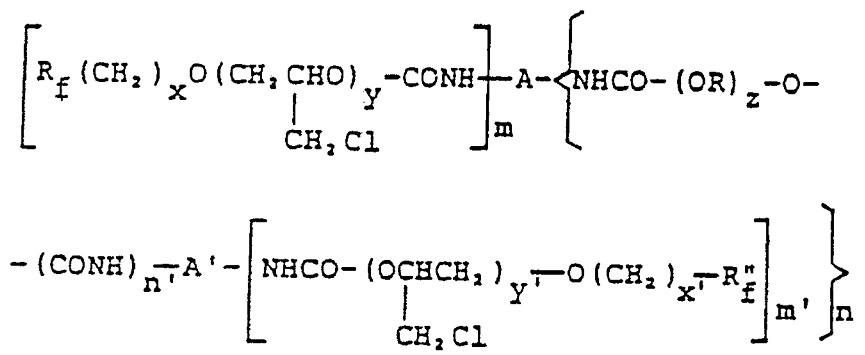 (14)

les divers symboles ayant les significations précédemment données.